(19) Europäisches Patentamt
European Patent Office
Office européen des brevets

(11) Numéro de publication: **0 327 426 B1**

(12) **FASCICULE DE BREVET EUROPEEN**

(45) Date de publication de fascicule du brevet: **03.11.93**

(51) Int. Cl.5: **C07D 498/22**, A61K 31/535, C07D 209/14, C07D 413/06, C07D 498/14, C07C 69/63, //(C07D498/22,265:00,221:00, 221:00,209:00)

(21) Numéro de dépôt: **89400203.9**

(22) Date de dépôt: **25.01.89**

(54) **Dérivés de l'indolo [3,2,1-de] [1,4]-oxazino [2,3,4-ij] [1,5]-naphthyridine, leur procédé de préparation et les intermédiaires ainsi obtenus, leur application comme médicaments et les compositions les renfermant.**

(30) Priorité: **26.01.88 FR 8800840**

(43) Date de publication de la demande:
**09.08.89 Bulletin 89/32**

(45) Mention de la délivrance du brevet:
**03.11.93 Bulletin 93/44**

(84) Etats contractants désignés:
**AT CH DE ES FR GB IT LI NL**

(56) Documents cités:
**EP-A- 0 061 768**
**FR-A- 2 372 804**
**FR-A- 2 383 182**

**JOURNAL OF ORGANIC CHEMISTRY, vol. 49, 1984, pages 2278-2279, American Chemical Society, Columbus, Ohio, US; J. VERCAUTEREN et al.: "Activated alkynes as partners in Pictet-Spengler condensations"**

(73) Titulaire: **ROUSSEL-UCLAF**
**35, Boulevard des Invalides**
**F-75007 Paris(FR)**

(72) Inventeur: **Aktogu, Nurgün**
**2, Rue Edmond About**
**F-92350 Le Plessis Robinson(FR)**
Inventeur: **Delevallée, Françoise**
**48-50, Avenue de la Dame Blanche**
**F-94120 Fontenay sous Bois(FR)**
Inventeur: **Clemence, François**
**2, Rue Turgot**
**F-75009 Paris(FR)**
Inventeur: **Oberlander, Claude**
**2, Rue Paul Albert**
**F-75018 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.6/3.3.1)

**CHEMICAL ABSTRACTS, vol. 77, 1972, page 457, résumé no. 48135q, Columbus, Ohio, US; I.D. PLETNEV et al.: "Indole derivatives. LXXV. Mono-beta-hydroxyethylation of tryptamines and synthesis of N,N'-bis[beta-(3-indolyl)-ethyl]ethylenediamines"**

**BEILSTEIN, vol. E III/IV, no. 22, page 4325**

(74) Mandataire: **Bourgouin, André et al**
**Département des Brevets**
**ROUSSEL UCLAF**
**111, route de Noisy**
**B.P. no 9**
**F-93230 Romainville (FR)**

## Description

L'invention concerne de nouveaux dérivés de l'indolo [3,2,1-de] [1,4]-oxazino [2,3,4-ij] [1,5] naphthyridine, leur procédé de préparation et les nouveaux intermédiaires ainsi obtenus, leur application comme médicaments et les compositions les renfermant.

Le brevet français BF 2.383.182 décrit des produits tétracycliques dérivés de la naphtyridine qui se distinguent des produits de la présente demande par l'absence d'un cycle D.

Dans le brevet européen EP 0.061.768 et les publications J. Org. Chem. 1984, 49, 2278-2279 et Chemical Abstracts 1972, 77, p. 457 48135 q, sont décrits des produits intermédiaires du même type structural que certains des intermédiaires de produits de la présente demande.

L'invention concerne de nouveaux composés de formule (I) :

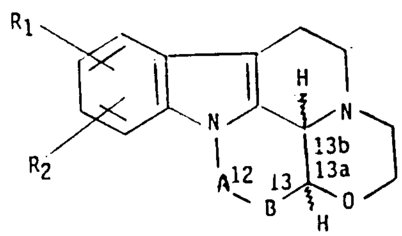

(I)

dans laquelle $R_1$ et $R_2$, identiques ou différents, représentent un atome d'hydrogène, un atome d'halogène, un radical alkyle ou alcoxy renfermant de 1 à 5 atomes de carbone, un radical hydroxy, trifluorométhyle ou nitro et dans laquelle le groupement :

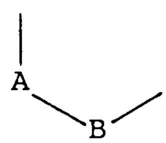

représente soit

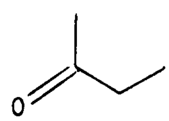

,

soit

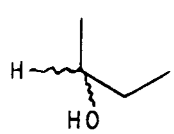

,

soit

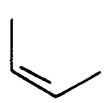

,

soit

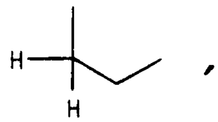

lesdits produits de formule (I) étant sous toutes les formes isomères possibles racémiques ou optiquement actives, ainsi que les sels d'addition avec les acides minéraux ou organiques desdits produits de formule (I).

Dans les produits de formule (I), l'atome d'hydrogène en position 13b et l'atome d'hydrogène en position 13a, peuvent chacun occuper l'une ou l'autre des orientations alpha et béta, ce qui détermine l'existence de diastéréoisomères cis et trans. De même, le radical hydroxy en position 12 peut être sous la forme alpha ou béta.

Lorsque $R_1$ et $R_2$ représentent un radical alkyle, il s'agit de préférence des radicaux méthyle, éthyle, n-propyle ou isopropyle mais ces substituants peuvent aussi représenter un radical n-butyle, isobutyle, n-pentyle.

Lorsque $R_1$ et $R_2$ représentent un radical alcoxy, il s'agit de préférence d'un radical méthoxy ou éthoxy, mais ils peuvent aussi représenter un radical propoxy, isopropoxy, butoxy linéaire, secondaire ou tertiaire.

Lorsque $R_1$ et $R_2$ représentent un atome d'halogène, il s'agit de préférence de l'atome de chlore, mais ils peuvent aussi représenter un atome de fluor, de brome ou d'iode.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, actique, formique, benzoïque, maléique, fumarique, succinique, tartrique, citrique, oxalique, glyoxylique, aspartique, ascorbique, les acides alcanemonosulfoniques, tels que l'acide méthanesulfonique, l'acide éthanesulfonique, l'acide propanesulfonique, les acides alcanedisulfoniques tels que l'acide méthanedisulfonique, l'acide alpha, béta-éthanedisulfonique, les acides arylmonosulfoniques, tel que l'acide benzènesulfonique et les acides aryldisulfoniques.

L'invention concerne notamment les composés de formule (I) caractérisés en ce que $R_1$ ou $R_2$ représente un atome d'hydrogène, un radical méthyle, éthyle, méthoxy ou éthoxy, un atome de chlorue, un radical hydroxy, trifluorométhyle ou nitro, sous toutes les formes isomères possibles racémiques ou optiquement actives, ainsi que les sels d'addition avec les acides minéraux ou organiques.

L'invention a plus particulièrement pour objet les composés de formule (I), caractérisés en ce que le groupement

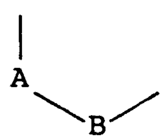

représente soit

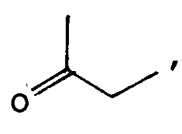

soit

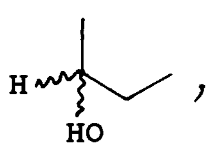

soit

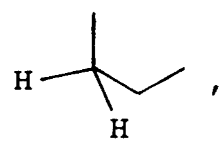

sous toutes les formes isomères possibles racémiques ou optiquement actives, ainsi que les sels d'addition avec les acides minéraux ou organiques.

L'invention concerne tout particulièrement :

- le [12RS (12alpha, 13a alpha, 13b béta)] (±) 2,3,5,6,12,13, 13a,13b-octahydro indolo [3,2,1-de] [1,4] oxazino [2,3,4-ij] [1,5] naphthyridin-12-ol,
- le [12RS (12alpha, 13a béta, 13b alpha)] (±) 3,5,6,12,13, 13a,13b-octahydro indolo [3,2,1-de] [1,4] oxazino [2,3,4-ij] [1,5] naphthyridin-12-ol,
- le (13aRS-trans) (±) 2,3,5,6,12,13,13a,13b-octahydro indolo [3,2,1-de] [1,4] oxazino [2,3,4-ij] [1,5] naphthyridine,
- le (13aRS-cis) (±) 2,3,5,6,13a,13b-hexahydro indolo [3,2,1-de] [1,4] oxazino [2,3,4-ij] [1,5] naphthyridin-12 (13H)-one, ainsi que leurs sels d'addition avec les acides minéraux ou organiques.

L'invention a aussi pour objet un procédé de préparation des composés de formule (I), caractérisé en ce que l'on fait réagir un composé de formule (II) :

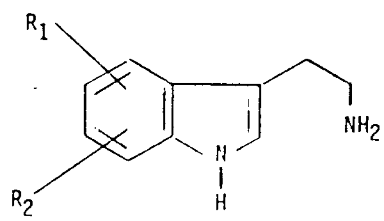

(II)

dans laquelle $R_1$ et $R_2$ ont les significations données ci-dessus avec un composé de formule :

$X\text{-}CH_2\text{-}COOR$

dans laquelle X est un atome d'halogène et R un radical alkyle renfermant 1 ou 2 atomes de carbone, pour obtenir un composé de formule (III) :

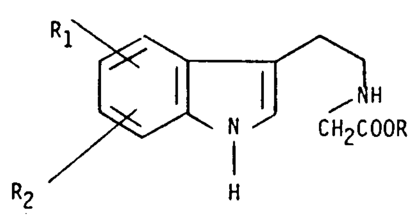

(III)

que l'on réduit pour obtenir un composé de formule (IV) :

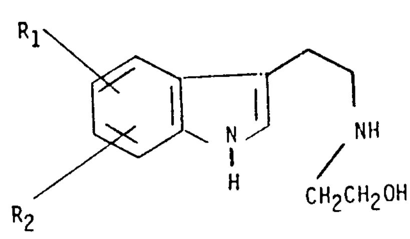

(IV)

que l'on condense

- soit avec l'anhydride maléique pour obtenir un produit de formule (V) :

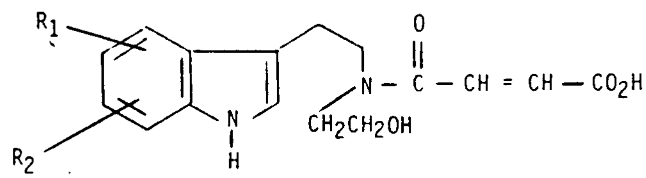

(V)

que L'on estérifie pour obtenir un produit de formule ($V_A$) :

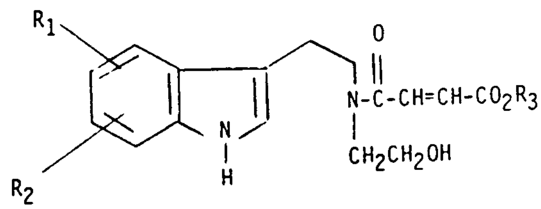

($V_A$)

dans laquelle $R_3$ est un radical alkyle renfermant 1 ou 2 atomes de carbone,

- soit avec un chlorure d'acide de formule (VI) :

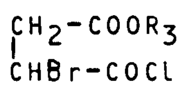

(VI)

dans laquelle $R_3$ est défini comme précédemment pour obtenir un mélange du produit de formule ($V_A$) tel que défini précédemment et du produit de formule (VII) :

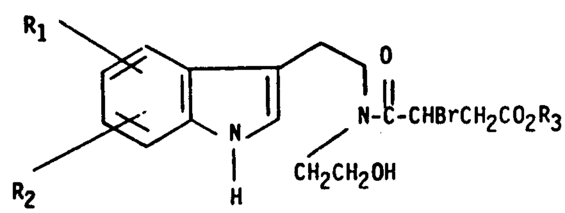

(VII)

produits de formule ($V_A$) et (VII) que l'on cyclise, pour obtenir un produit de formule (VIII) :

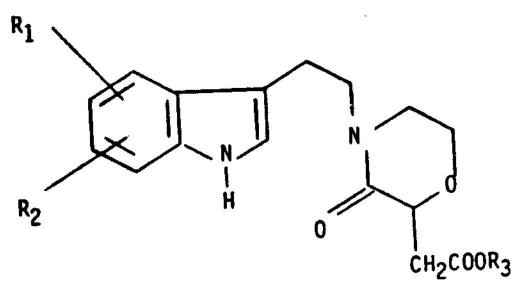

(VIII)

sur lequel l'on fair agir l'oxychlorure de phosphore, pour obtenir le produit de formule ($IX_1$) :

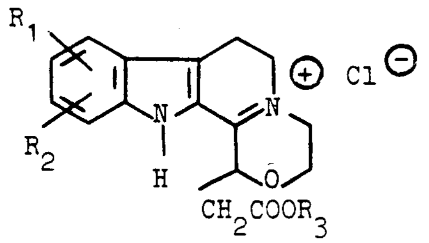

(IX$_1$)

que

- ou bien l'on transforme en perchlorate de formule (IX$_2$) :

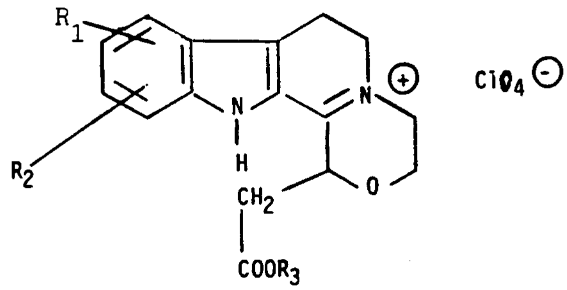

(IX$_2$)

que, soit l'on réduit le composé de formule (IX$_2$) en composé de formule (X) dans laquelles les deux atomes d'hydrogène en positions 13b et 13a sont en position cis :

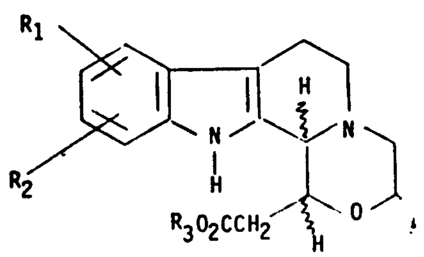

(X)

que l'on cyc lise en milieu acide, pour obtenir un composé de formule (IA$_1$) correspondant à un produit de formule (I) dans laquelle :

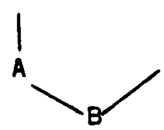

représente

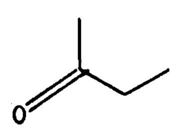

et les deux atomes d'hydrogène en position 13b et 13a sont en position cis
soit l'on cyclise en milieu acide le composé de formule (IX$_2$) en composé de formule (XI) :

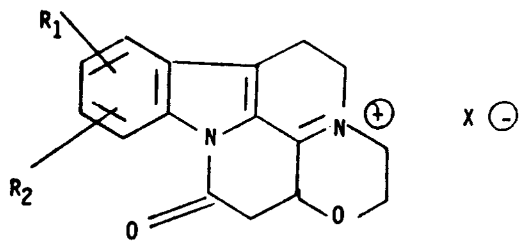

(XI)

$X^-$ étant l'anion provenant de l'acide utilisé et réduit,
pour obtenir un composé de formule ($IA_2$) correspondant à un produit de formule (I) dans laquelle

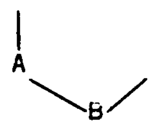

représente

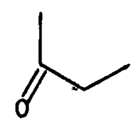

et les deux atomes d'hydrogène en position 13b et 13a sont en positions trans,

- ou bien l'on cyclise le produit de formule ($IX_1$) en milieu acide ou en milieu basique, pour obtenir un produit de formule (XII) :

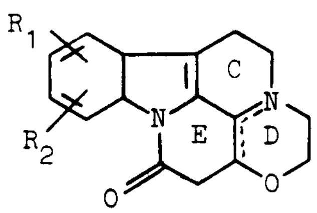

(XII)

dans laquelle le trait pointillé représente une double liaison entre les cycles C et D ou entre les cycles D et E, que l'on réduit, pour obtenir un produit de formule ($IA_2$) telle que définie précédemment, lesdits composés de formule ($IA_1$) et ($IA_2$) étant, si désiré, réduits en composés correspondants de formules ($IB_1$) et ($IB_2$), représentant les composés de formule (I) dans laquelle

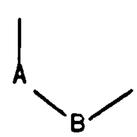

représente

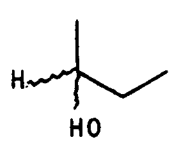

lesdits composés de formules ($IB_1$) et ($IB_2$) étant, si désiré, déshydratés, pour obtenir des composés

correspondants de formule ($IC_1$) et ($IC_2$), représentant les composés de formule (I) dans laquelle

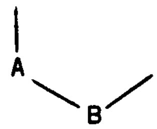

représente

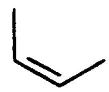

lesdits composés de formules ($IC_1$) et ($IC_2$) étant, si désiré, réduits en composés correspondants de formule ($ID_1$) et ($ID_2$) représentant les composés de formule (I) dans laquelle

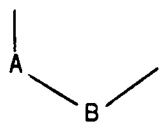

représente

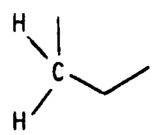

et salifie, si désiré, les produits de formule (I) obtenus par action d'un acide minéral ou organique.

Dans des conditions préférentielles de mise en oeuvre de l'invention, le procédé ci-dessus est réalisé de manière suivante :

- Le composé réagissant avec le composé de formule (II) est le bromo acétate d'éthyle, la réaction est réalisée en présence d'une base comme la triéthylamine ou le carbonate de potassium.
- La réduction du composé de formule (III) en composé de formule (IV) est réalisée avec un hydrure mixte, tel que par exemple l'hydrure mixte de lithium et d'aluminium, le diéthylhydrure de sodium et d'aluminium, le triéthylhydrure de bore et de lithium.
- La réaction du composé de formule (IV) avec l'anhydride maléique ou avec le composé de formule (VI) est réalisée par transfert de phase. On utilise comme phase organique, un solvant tel que le chloroforme ou le mélange benzèneacétonitrile. On opère en présence d'une base comme le carbonate de potassium et d'un agent de transfert de phase, notamment le bromure ou l'hydrogéno-sulfate de tétrabutylammonium.
- $R_3$ est de préférence un radical méthyle dans le composé de formule ($V_A$) et dans le composé de formule (VI).
- L'estérification du composé de formule (V) en composé de formule ($V_A$) est réalisée avec une résine échangeuse d'ions dans le méthanol au reflux.
- La cyclisation des composés de formules ($V_A$) et (VII) en composé de formule (VIII) s'effectue en présence de carbonate de potassium.
- Le perchlorate de formule ($IX_2$) est obtenu par action de l'acide perchlorique en milieu aqueux.
- La réduction du composé de formule ($IX_2$) en composé de formule (X) s'effectue avec du borohydrure de sodium.
- La cyclisation du composé de formule (X) en composé de formule ($IA_1$) est réalisée en présence d'acide chlorhydrique.
- La cyclisation du composé de formule ($IX_2$) en sel d'immonium de formule (XI) est réalisée par l' acide chlorhydrique ou iodhydrique.

- La cyclisation du composé de formule ($IX_1$) est réalisée soit dans des conditions telles que celles décrites précédemment conduisant au composé de formule (XI) et dans ce cas, la double liaison est placée entre les cycles C et D, soit à l'aide d'une base, par exemple l'ammoniaque, en présence d'un antioxydant tel que l'acide ascorbique, et dans ce cas, la double liaison est placée entre les cycles D et E.
- La réduction des composés de formules (XI) et (XII) en composé de formule ($IA_2$) est effectuée par du borohydrure de sodium en présence d' acicd acétique.
- La réduction des composés de formule ($I_A$) dans laquelle

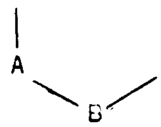

représente

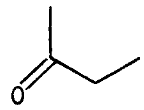

en composés de formule ($I_B$) dans laquelle

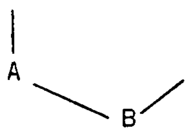

représente

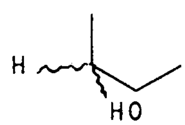

est réalisée avec un hydrure, notamment un hydrure mixte, tel que par exemple, l'hydrure mixte de lithium et d'aluminium, le diéthylhydrure de sodium et d'aluminium.
- L'agent de déshydratation utilisé pour obtenir à partir des composés de formule ($I_B$) dans laquelle

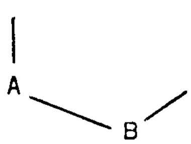

représente

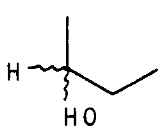

des composés de formule ($I_C$) dans laquelle

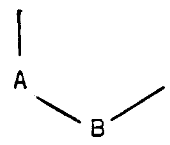

représente

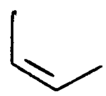

est un acide tel que l'acide chlorhydrique, l'acide bromhydrique, l'acide sulfurique, l'acide acétique, l'acide para-toluènesulfonique, l'acide méthanesulfonique en quantité catalytique.

- L' agent de réduction auxquels sont soumis les composés de formule ($I_C$) dans laquellle

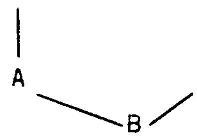

représente

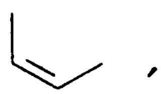

pour obtenir les composés de formule ($I_D$) dans laquelle

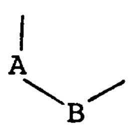

représente

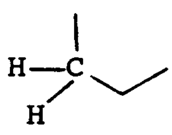

est l'hydrogène en présence d'un catalyseur tel que le platine, le palladium.

- Les formes optiquement actives des produits de formule (I) peuvent être préparées par dédoublement des racémiques, selon les méthodes usuelles.

Les composés de formule (I) tels que définis ci-dessus ainsi que leurs sels d'addition avec les acides présentent d'intéressantes propriétés pharmacologiques. Ils présentent en particulier une très bonne activité analgésique.

Ils présentent également des propriétés anti-dépressives, nootropes (notamment anti-amnésiantes), protectrices neuronales, anti-anoxiques et anti-ischémiques intéressantes.

Ces propriétés justifient leur application en thérapeutique et l'invention a également pour objet à titre de médicaments, les produits tels que définis par la formule (I) ci-dessus, lesdits produits de formule (I) étant sous toutes les formes isomères possibles racémiques ou optiquement actives ainsi que les sels d'addition

avec les acides minéraux ou organiques pharmaceutiquement acceptables desdits produits de formule (I).

L'invention a plus particulièrement pour objet, à titre de médicaments :

- le [12RS (12alpha, 13a alpha, 13b béta)] (±) 3,5,6,12,13, 13a,13b-octahydro indolo [3,2,1-de] [1,4] oxazino [2,3,4-ij] [1,5] naphthyridin-12-ol,
- le [12RS (12alpha, 13a béta, 13b alpha)] (±) 2,3,5,6,12,13, 13a,13b-octahydro indolo [3,2,1-de] [1,4] oxazino [2,3,4-ij] [1,5] naphthyridin-12-ol,
- le (13aRS-trans) (±) 2,3,5,6,12,13,13a,13b-octahydro indolo [3,2,1-de] [1,4] oxazino [2,3,4-ij] [1,5] naphthyridine,
- le (13aRS-cis) (±) 2,3,5,6,13a,13b-hexahydro indolo [3,2,1-de] [1,4] oxazino [2,3,4-ij] [1,5] naphthyridin-12 (13H)-one, et leurs sels pharmaceutiquement acceptables.

Les médicaments, objet de l'invention, peuvent être utilisés dans le traitement des algies musculaires, articulaires ou nerveuses, des douleurs dentaires, des migraines, du zona et également à titre de' traitement complementaire dans les états infectieux et fébriles.

Ils peuvent être aussi utilisés dans le traitement des insuffisances cérébrales d'origine anoxique ou ischémique, des troubles de la mémoire et de l'attention. Ils peuvent également être utilisés comme anti-dépresseurs.

L'invention s'étend aux compositions pharmaceutiques renfermant comme principe actif, les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme, par exemple, les comprimés simples ou dragéifiés, les gélules, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple de 10 mg à 2 g par jour chez l'adulte, par voie orale.

La présente invention a enfin pour objet les produits de formules (V), ($V_A$), (VII), (VIII), ($IX_1$), ($IX_2$), (X) et (XII) telles que définies ci-dessus.

Les chlorures d'acides de formule (VI) sont préparés de façon usuelle par chloruration des acides correspondants.

Les exemples donnés ci-après illustrent l'invention.

**Exemple 1 : Chlorhydrate de (13aRS-cis) (+) 2,3,5,6,13a, 13b-hexahydro indolo [3,2,1-de] [1,4] oxazino [2,3,4-ij] [1,5] naphthyridin-12 (13H)-one (cis dl).**

**Stade A :** [[2-(1H-indol-3-yl) éthyl] amino] acétate d'éthyle.

On agite une heure 500 g de tryptamine dans 11 litres de chlorure de méthylène, ajoute en une seule fois 512 g de carbonate de potassium dans 1540 cm3 d'eau puis en une seule fois 350 cm3 de bromoacétate d'éthyle. On agite vigoureusement le mélange réactionnel pendant 20 heures. On décante, extrait la phase aqueuse au chlorure de méthylène, lave l'ensemble des phases organiques avec de l'eau, réextrait les lavages aqueux par du chlorure de méthylène. On sèche les phases organiques réunies et amène à sec à 40¤C sous 20 mm de pression. On chromatographie le résidu sur silice, élue d'abord par un mélange chlorure de méthylène-acétate d'éthyle (8-2) pour obtenir 155,3 g de diester puis par de l'acétate d'éthyle pur pour obtenir 398,5 g de produit attendu utilisé tel que dans la suite de la synthèse.

**Stade B :** 2-[[2-(1H-indol-3-yl) éthyl] amino] éthanol.

On dissout 398 g de produit obtenu ci-dessus dans 4 litres de tétrahydrofuranne, refroidit à +10¤C et introduit, en maintenant cette température, 400 g de diéthyl dihydroaluminate de sodium à 25% dans le toluène. On agite 1 heure 30 minutes en laissant remonter la température à 17¤C. On refroidit le milieu à -10¤C et introduit en 1 heure 2u minutes 400 cm3 de lessive de soude dans 1200 cm3 d'eau en limitant la température à 0¤C pendant l'introduction. On agite 2 heures à +10¤C puis laisse remonter à température ambiante pandant 16 heures en maintenant une agitation plus faible. On décante, lave la phase organique à

l'eau, extrait les lavages à l'acétate d'éthyle et au chlorure de méthylène. On réunit les phases organiques, dilue au chlorure de méthylène, sèche, chasse les solvants à 40¤C sous pression réduite. On empâte le résidu dans 1300 cm3 d'acétate d'éthyle pendant 1 heure, essore, lave à l'acétate d'éthyle, sèche et obtient 240,8 g de produit attendu. F = 92¤C.

**Stade C :** [4-[2-(1H-indol-3-yl) éthyl] 3-oxo 2-morpholinyl] acétate de méthyle.

On met en suspension 4,08 g de produit obtenu ci-dessus dans 90 cm3 de mélange benzène-acétonitrile (2-1), ajoute 5,5 g de carbonate de potassium dans 30 cm3 d'eau puis 0,4 g de bromure de tétrabutylammonium. On agite fortement le milieu pour ajouter 1,930 g d'anhydride maléique et chauffe 24 heures au reflux sous forte agitation. Après refroidissement, on acidifie par une solution d'acide chlorhydrique 2N jusqu'à pH 3-4 et décante les deux phases. La solution aqueuse est réextraite par un mélange chloroforme-méthanol (8-2). On rassemble les solutions organiques, sèche et concentre à sec. On récupère 6,1 g de produit. On dissout 4,5 g de ce dernier dans 90 cm3 de méthanol, ajoute 4,5 g de résine échangeuse d'ions et chauffe 20 heures au reflux. Après refroidissement, on essore la résine, la lave au méthanol, concentre le filtrat et les lavages sous pression réduite et obtient 3,4 g de produit huileux. On dissout 1,7 g de ce dernier dans 34 cm3 de tétrahydrofuranne, ajoute 1,7 g de carbonate de potassium, chauffe au reflux sous atmosphère inerte pendant 16 heures. On filtre, lave à l'acétate d'éthyle et concentre à sec le filtrat. On récupère 0,900 g de produit attendu. F = 144¤C.

**Stade D :** Perchlorate de 1-(2-méthoxy 2-oxoéthyl) 1,3,4,6, 7, 12-hexahydro [1,4] oxazino [4′,3′-1,2] pyrido [3,4-b] indol-5-ium.

On met en suspension 5 g de produit obtenu au stade C dans 50 cm3 d'oxychlorure de phosphore et chauffe 3 heures au reflux. On concentre jusqu'à 25 cm3 environ puis coule lentement en 15 minutes dans 250 cm3 de glace et eau contenant 25 cm3 d'acide perchlorique à 65%. On agite 1 heure à 0¤C, essore, lave à l'eau, sèche sous pression réduite à 30¤C et obtient 5,5 g de produit attendu utilisé tel quel pour la suite des opérations.

**Stade E :** (1R,cis) (±) 3,4,5,6,11,11b-hexahydro 1H-[1,4] oxazino [4',3',1,2] pyrido [3,4-b] indol-1-acétate de méthyle.

On dissout le perchlorate encore humide dans 300 cm3 de mélange méthanol-tétrahydrofuranne (1-1), refroidit à 5-10¤C, ajoute en 15 minutes 3,6 g d'hydroborure de sodium, agite pendant 1 heure puis concentre à sec sous pression réduite. On empate le résidu dans 150 cm3 d'eau pendant 30 minutes, essore, lave à l'eau et reprend dans 300 cm3 de mélange chloroforme-méthanol (2-1). On essore l'insoluble et concentre à sec le filtrat. On cristallise le résidu dans le méthanol, chromatographie 2,70 g de produit sur silice, élue par un mélange chlorure de méthylène-méthanol (9-1) et isole 2,35 g de produit attendu. Après cristallisation dans le méthanol, le produit fond à 207¤C.

**Stade F :** Chlorhydrate de (13aRS cis) (+) 2,3,5,6,13a,13b-hexahydro indolo [3,2,1-de] [1,4] oxazino [2,3,4-ij] [1,5] naphthyridin-12 (13H)-one (cis dl).

On met en suspension 8,9 g de produit obtenu ci-dessus dans 89 cm3 d'acide chlorhydrique 5N et chauffe au reflux pendant 3 heures. On refroidit à 0¤/-5¤C et amène à pH alcalin par addition d'ammoniaque concentrée, agite 1 heure à 0¤/-5¤C. On essore, lave à l'eau, sèche sous pression réduite à 60¤C et obtient 7,5 g de produit. F = 205¤C. On dissout partiellement 5 g de ce dernier au reflux dans 125 cm3 d'éthanol, ajoute à chaud 1,7 cm3 d'acide chlorhydrique concentré. On traite la solution au charbon actif, filtre, refroidit à 0¤C et essore le chlorhydrate que l'on recristallise sous séchage dans 200 cm3 de méthanol. On obtient 3,94 g de produit attendu.

Spectre RMN 250 MHz ppm

3,66-3,78 : H en 2
4,08-4,16 : H en 13a - 13b
2,43-2,70 : H en 5
2,54-2,90 : H en 6
2,96 : H en 13

7,44-8,38 : H en 7 et 10
7,30 : H en 8-9

**Exemple 2 : Chlorhydrate de (13aRS,trans) (±) 2,3,5,6,13a, 13b-hexahydro indolo [3,2,1-de] [1,4] oxazino [2,3,4-ij] [1,5] naphthyridin-12 (13H)-one (trans dl).**

**Stade A :** Chlorure de 3,5,6,12,13,13a-hexahydro 12-oxo 2H-indolo [3,2,1-de] [1,4] oxazino [2,3,4-ij] [1,5] naphthyridin4-ium.

On met en suspension 4,4 g de perchlorate de 1-(2-méthoxy 2-oxoéthyl) 1,3,4,6,7,12-hexahydro [1,4] oxazino [4',3'-1,2] pyrido [4,4-b] indol-5-ium dans 38 cm3 d'acide chlorhydrique 5N et chauffe à 100¤C pendant 16 heures. On refroidit le mélange réactionnel à 0¤C pendant 30 minutes, essore le sel, le lave avec de l'eau, le sèche et l'utilise aussitôt au stade suivant.

**Stade B :** Chlorhydrate de (13aRS,trans) (±) 2,3,5,6,13a, 13b-hexahydro indolo [3,2,1-de] [1,4] oxazino [2,3,4-ij] [1,5] naphthyridin-12 (13H)-one (trans dl).

On dissout partiellement le sel encore humide obtenu au stade A dans 100 cm3 d'acide acétique, refroidit à 10¤C, ajoute en 25 minutes 3 g de borohydrure de sodium en maintenant la température inférieure à 20¤C. On agite 1 heure puis distille l'acide acétique. On reprend le résidu par 150 cm3 d'eau, refroidit à 0¤/5¤C, alcalinise en 30 minutes avec de l'ammoniaque concentrée, agite 1 heure à 0¤/5¤C, essore, lave à l'eau, sèche sous pression réduite à 70¤C. On chromatographie le produit sur silice, élue par un mélange chlorure de méthylène-méthanol (9-1) et isole 7 g de produit sous forme de base. On recristallise 2 g de ce dernier dans du méthanol. On dissout le produit obtenu dans 70 cm3 de méthanol au reflux et ajoute 1 cm3 d'acide chlorhydrique concentré. On agite 30 minutes à 0¤/5¤C, essore, lave et sèche sous pression réduite à 50¤C. On obtient 1,90 g de produit attendu. F = 230¤C.

Spectre RMN 250 MHz $CDCl_3$ ppm

3,73 (d,d,d J = 5,9-11,5) : CH-O jonction trans
3,92 à 4,10 : $CH_2O$
7,25 à 7,35 : (2H) aromatiques
7,42 (m) : (1H)
8,32 (m) : (1H)
2,63 à 3,22 : (9H) autres protons.

**Exemple 3 : [12RS (12alpha, 13a alpha, 13b alpha)] (±) 2,3, 5,6,12,13,13a,13b-octahydro indolo [3,2,1-de] [1,4] oxazino [2,3,4-ij] [1,5] naphthyridin-12-ol (cis dl).**

On dissout 8 g de chlorhydrate de (13aRS,cis) (±) 2,3,5, 6,13a,13b-octahydro indolo [3,2,1-de] [1,4] oxazino [2,3,4-ij] [1,5] naphthyridin-12 (13H)-one dans 400 cm3 de tétrahydrofuranne, refroidit à 0¤/5¤C sous atmosphère inerte, ajoute 25,5 cm3 d'une solution toluénique de dihydrure de diéthylaluminium-sodium titrée à 0,236 m% cm3) et agite pendant 1 heure sous les conditions ci-dessus. En maintenant à 0¤/5¤C, on hydrolyse le milieu par 200 cm3 de tétrahydrofuranne à 25% d'eau puis ajoute 100 cm3 d'eau et 100 cm3 d'acétate d'éthyle. On décante, extrait la phase aqueuse par 100 cm3 d'éthyle, lave avec de l'eau. On extrait les eaux de lavage avec de l'acétate d'éthyle. On sèche les phases organiques réunies, concentre à sec. On chromatographie sur silice le résidu, élue par un mélange chlorure de méthylène-méthanol (9-1) et isole 2,2 g de produit avec OH axial et 4,1 g de produit avec OH équatorial. On recristallise ce dernier dans l'isopropanol. F = 206¤C.

Spectre RMN 250 MHz $CDCl_3$ ppm

5,58 : H en 12
3,84-3,95 : H en 13a et 13b cis
3,51 à 3,68 : H en 2
2,30 : H en 3
3,25 : H en 6
1,73 : H en 13

7,17-7,48-7,74 : aromatiques
2,5 à 3,1 : autres protons.

**Exemple 4 : [12RS (12alpha, 13a béta, 13b béta)] (±) 2,3,5, 6,12,13,13a,13b-octahydro indolo [3,2,1-de] [1,4] oxazino [2,3,4-ij] [1,5] naphthyridin-12-ol (cis dl).**

On opère comme à l'exemple 3 et obtient les 2 épimères 2,2 g avec OH axial et 4,1 g avec OH équatorial. On recristallise l'épimère à OH axial dans l'isopropanol au reflux et obtient 1,5 g de produit attendu. F = 180¤C.

Spectre RMN 250 MHz $CDCl_3$ ppm

| | | |
|---|---|---|
| 4,09-4,25 | : H en 13a et 13b cis | |
| 5,92 | : H en 12 équatorial | |
| 3,97 | : OH axial | |
| 3,67-3,83 | : H en 2 | |
| 2,46-2,80 | : H en 3 | |
| 3,35 | : H en 6 | |
| 7,51 | : (2H) | |
| 7,1 à 7,25 | : 2H aromatiques | |
| 3,00 | : (1H) | autres protons |
| 2,65 | : (2H) | |

**Exemple 5 : [12RS (12alpha, 13a béta, 13b alpha)] (±) 2,3,5, 6,12,13,13a,13b-octahydro indolo [3,2,1-de] [1,4] oxazino [2,3,4-ij] [1,5] naphthyridin-12-ol (trans dl).**

On dissout 0,8 g de [13aRS,trans) (+) 2,3,5,6,13a,13b-hexahydro indolo [3,2,1-de] [1,4] oxazino [2,3,4-ij] [1,5] naphthyridin-12 (13H)-one dans 20 cm3 de tétrahydrofuranne anhydre. On refroidit sous atmosphère inerte à -70¤C, introduit en 15 minutes, 2,55 cm3 d'une solution toluénique de dihydrure de diéthylaluminium-sodium titré à 23,6%, agite 1 heure dans ces conditions. A -20¤C, on hydrolyse le milieu réactionnel par 10 cm3 de tétrahydrofuranne à 25% d'eau. On décante, réextrait la phase aqueuse à l'acétate d'éthyle, lave les phases organiques réunies à l'eau jusqu'à pH neutre, sèche et concentre à sec sous pression réduite. On recristallise le résidu dans 60 cm3 d'isopropanol au reflux et récupère 0,600 g d'un mélange à 95% de produit à OH équatorial et 5% d'épimère à OH axial. Après une 2ème cristallisation, on obtient le produit OH équatorial attendu pur. F = 225¤C.
Rf. = 0,47 (système acétone - $CH_2Cl_2$ 1-1 pour produit à Rf. = 0,35 OH axial)

Spectre RMN 250 MHz $CDCl_3$ ppm

5,63 (dt J = 5,5 et 9) : H en 12
6,68 (d, J = 9) : OH
7,0 à 7,15 : H5 et H6 indole
7,39 à 7,65 : H4 et H7 indole
1,9 à 4,0 : autres protons.

**Exemple 6 : [12RS (12alpha, 13a alpha, 13b béta)] (±) 2,3,5, 6,12,13,13a,13b-octahydro indolo [3,2,1-de] [1,4] oxazino [2,3,4-ij] [1,5] naphthyridin-12-ol (trans dl).**

On opère comme à l'exemple 4 et obtient 9 g d'un mélange d'épimères (OH axial et équatorial), 3 g du mélange sont mis en suspension dans 30 cm3 d'acide chlorhydrique 2N, chauffés 48 heures à 40¤C. On refroidit et neutralise par 6 cm3 d'ammoniaque concentrée. On essore puis recristallise dans 200 cm3 de méthanol et obtient 1,5 g d'épimère OH axial. F = 250¤C. Des eaux mères, on récupère 1 g de produit

attendu. Rf. = 0,35 (système $CH_2Cl_2$-acétone 1-1).

Spectre RMN 250 MHz DMSO ppm

6,05 (m) : H en 12
6,40 (d, J = 6,5) : OH axial
7,01 (dt) 7,08 (dt) : $H_5$-$H_6$ indole
7,38 (dd) 7,45 (dd) : $H_4$-$H_7$ indole
3,95 (dd) : H en 2 (équatorial)
3,77 (m) : H en 2 (axial) et H de jonction en 13a
2,65 à 3,1 : autres protons.

**Exemple 7 : Hémifumarate de [13aRS,cis) (±) 2,3,5,6,13a,13b-hexahydro indolo [3,2,1-de] [1,4] oxazino [2,3,4-ij] [1,5] naphthyridine.**

On met en suspension dans 40 cm3 de tolène 4,1 g du mélange des 2 épimères hydroxylés obtenu à l'exemple 3, chauffe jusqu'à 80¤C pour dissoudre le produit et introduit 0,2 g d'acide paratoluènesulfonique. On chauffe au reflux pendant 16 heures, refroidit et filtre. On lave la phase organique avec 20 cm3 d'ammoniaque concentrée diluée au demi puis avec 20 cm3 d'eau. On extrait les eaux de lavage au toluène. On réunit les phases organiques, sèche et concentre à sec. On empâte le résidu dans 20 cm3 d'éther isopropylique, essore et sèche sous pression réduite à 50¤C. On obtient 3 g de produit. F = 148¤C. On dissout 2 g de ce dernier dans 30 cm3 d'acétate d'éthyle au reflux, ajoute en une seule fois une solution bouillante de 0,465 g d'acide fumarique dans 15 cm3 d'isopropanol, agite 30 minutes à 0¤C, essore, sèche à 70¤C sous pression réduite et obtient 2,12 g de produit attendu. F = 245¤C.

**Exemple 8 : Fumarate de (13aRS,trans) (±) 2,3,5,6,13a,13b-hexahydro indolo [3,2,1-de] [1,4] oxazino [2,3,4-ij] [1,5] naphthyridine.**

On opère comme à l'exemple 7 à partir de 4,3 g du mélange 2u des 2 épimères hydroxylés obtenu à l'exemple 5 et obtient 3,4 g de produit sous forme de base. F = 158¤-169¤C. On dissout 3,3 g de ce dernier dans 50 cm3 d'acétate d'éthyle, ajoute en une fois 0,600 g d'acide fumarique, dissout dans 75 cm3 d'acétate d'éthyle bouillant. On refroidit à -50¤C et laisse 1 heure à cette température. On essore, lave à l'acétate d'éthyle glacé, sèche et obtient 1,2 g de fumarate acide attendu. F = 183-186¤C.

**Exemple 9 : Fumarate de (13aRS,trans) (±) 2,3,5,6,12,13,13a, 13b-octahydro indolo [3,2,1-de] [1,4] oxazino [2,3,4-ij] [1,5] naphthyridine.**

On dissout 3,8 g de produit obtenu à l'exemple 8 dans 190 cm3 d'éthanol, ajoute 0,380 g de dioxyde de platine à 82% et effectue l'hydrogénation pendant 1 heure 30 minutes. On filtre, évapore le filtrat et obtient 3,9 g de produit saturé. F = 125¤C. On dissout 2 g de ce dernier dans 60 cm3 d'un mélange d'acétate d'éthyle-éther isopropylique (1-1). On filtre, ajoute au filtrat 0,460 g d'acide fumarique, dissout dans 20 cm3 d'éthanol bouillant. On refroidit à -15¤/-10¤C pendant 1 heure. On essore, lave par un mélange acétate d'éthyle-éther isopropylique glacé, sèche et obtient 1,37 g de fumarate acide attendu. F = 210¤C.

Spectre RMN de la base 400 MHz $CDCl_3$ jonction trans.

3,50 (d,d,d J = 4,5-9-11,5) : CH-O axial
3,08 : CH-N axial
4,34 (m) 3,80 (m) : $CH_2$-N indole
3,98 (dt)4,06 (m) : $CH_2$-O morpholine

**Exemple 10 : Hémifumarate de (13aRS,cis) (±) 2,3,5,6,12,13, 13a,13b-octahydro indolo [3,2,1-de] [1,4] oxazino [2,3,4-ij] [1,5] naphthyridine (cis dl).**

On dissout 3,30 g de produit obtenu à l'exemple 7 dans 200 cm3 d'éthanol, ajoute 0,33 g d'oxyde de platine à 82% et effectue l'hydrogénation pendant 1 heure 15 minutes. On filtre, concentre le filtrat jusqu'à 100 cm3 et ajoute 0,755 g d'acide fumarique dissous dans 25 cm3 d'éthanol bouillant. On agite 1 heure à

température ambiante, essore et obtient 3,30 g d'hémifumarate attendu. F = 240¤C.

Spectre RMN $CDCl_3$ ppm sur base

4,08 (jonction cis) : CH-N et CH-O
4,12 (m) : $CH_2N$ indole
7,12 (dt) 7,19 (dt) : $H_5$ et $H_6$ indole
7,32 (d) 7,50 (d) : $H_4$ et $H_7$ indole
2,1 à 3,9 : autres protons.

**Exemple 11 : Maléate acide de (16alpha) (±) 11-méthoxy 20, 21-dinor 17-oxaéburnaménin-14 (15H)-one.**

**Stade A :** [2-[(6-méthoxy 1H-indol-3-yl) éthyl] amino] acétate d'éthyle.

A une suspension de 19 g de 6-méthoxy tryptamine et 380 cm3 de tétrahydrofuranne, on ajoute 18,3 g de 1,8-diazabicyclo [5.4.0.] undec-7-ène (DBU) à la solution obtenue, on ajoute encore 20 g de bromoacétate d'éthyle et agite 24 heures à température ambiante. On filtre l'insoluble et concentre le filtrat à sec sous pression réduite. Le résidu (20 g) est chromatographié sur silice (éluant : chlorure de méthylèneméthanol 95-5), on obtient 19 g de produit recherché. F ≃ 60¤C.

Spectre IR ($CHCl_3$)

NH indole : 3479 $cm^{-1}$
NH-$CH_2$ : 3322 $cm^{-1}$
>C=O : 1734 $cm^{-1}$

**Stade B :** 2-[2-[(6-méthoxy 1H-indol-3-yl) éthyl] amino] éthanol.

A une solution de 20 g du produit obtenu au stade A dans 400 cm3 de tétrahydrofuranne, on ajoute à 20¤C ± 3¤C, 245 cm3 d'une solution 2,3M de diéthylhydrure de sodium et d'aluminium dans le toluène et on agite pendant 2 heures à température ambiante. On ajoute ensuite lentement à 20¤C ± 3¤C, 200 cm3 de tétrahydrofuranne à 20% d'eau, on agite 1 heure 30 minutes à température ambiante. On filtre l'insoluble qui est lavé par deux fois 400 cm3 de tétrahydrofuranne. Le filtrat et les lavages sont concentrés à sec sous pression réduite. On empâte le résidu dans 200 cm3 d'éther isopropylique et obtient 13,5 g de produit recherché. F = 123¤C.

Spectre IR ($CHCl_3$)

OH : 3610 $cm^{-1}$
NH indole : 3480 $cm^{-1}$
aromatique : 1630, 1580, 1558, 1501 $cm^{-1}$

**Stade C :** [4-[2-(6-méthoxy 1H-indol-3-yl) éthyl] 3-oxo 2-morpholinyl] acétate de méthyle.

A une suspension de 13,4 g d'amino alcool obtenu au stade B et 165 cm3 de chloroforme, on ajoute une solution de 39,5 g de carbonate de potassium dans 80 cm3 d'eau et 0,67 g de bromure de tétrabutylammonium. On introduit en 45 minutes à température ambiante 0,063 mole de chlorure d'acide 2-bromo 4-méthyl butanedioïque (préparation 1) en solution dans 165 cm3 de chloroforme, on agite 20 heures à température ambiante. On sépare les phases par décantation et réextrait la phase aqueuse par deux fois 100 cm3 de chloroforme. Les fractions organiques lavées à l'eau sont concentrées à sec sous pression réduite. Le résidu (25 g) est mis en solution dans 250 cm3 de tétrahydrofuranne, on ajoute 25 g de carbonate de potassium et agite 16 heures au reflux. On filtre l'insoluble, le lave par trois fois 100 cm3 de tétrahydrofuranne et concentre le filtrat et les lavages à sec sous pression réduite. On chromatographie le résidu (25 g) sur silice (éluant : acétate d'éthyle-chlorure de méthylène 8-2) et obtient 16 g de produit recherché.

Spectre IR ($CHCl_3$)

carbonyle : 1735 $cm^{-1}$ ester, 1649 $cm^{-1}$
$CH_3$ du $COOCH_3$ : 1439 $cm^{-1}$
aromatiques : 1571, 1558, 1500, 1493 $cm^{-1}$
$OCH_3$ : 2835 $cm^{-1}$

**Stade D :** (16alpha) (±) 11-méthoxy 20,21-dinor 17-oxaéburnaménin-14 (15H)-one.

A une solution de 14 g du produit obtenu au stade C dans 280 cm3 de dioxanne, on ojoute 14 cm3 d'oxychlorure de phosphore et agite 3 heures à 80¤C. On amène à sec sous pression réduite. On reprend le résidu avec 140 cm3 d'acétone, ajoute 1,4 g d'acide ascorbique, chauffe à 45-50¤C et introduit à cette température 140 cm3 d'ammoniaque concentrée, on agite pendant 1 heure 30 minutes à 50¤C. On ajoute au milieu réactionnel 560 cm3 d'eau et glace et agite 1 heure 30 minutes. Le solide précipité est isolé par essorage et lavé à l'eau glacée. Le produit humide est repris avec 280 cm3 d'acide acétique et réduit par ajout à 15¤C ± 5¤C, de 14 g de borohydrure de sodium, on agite pendant 1 heure et alcalinise le milieu, à 15¤C ± 5¤C, par ajout d'ammoniaque concentrée. On essore la suspension et chromatographie le produit brut (10 g) sur silice (éluant : chlorure de méthylène-méthanol 95-5) puis une nouvelle fois avec acétate d'éthyle seul. On obtient 6 g de produit attendu. F = 164¤C.

Spectre IR ($CHCl_3$)

>=0 : 1706 $cm^{-1}$
aromatique : 1663, 1616, 1575, 1487 $cm^{-1}$
bandes de Bohlmann.

**Stade E :** Maléate acide de (16alpha) (±) 11-méthoxy 20,21-dinor 17-oxaéburnaménin-14 (15H)-one.

On dissout 0,800 g du produit obtenu au stade D dans 20 cm3 d'acétate d'éthyle bouillant et ajoute 0,310 g d'acide maléique en solution dans 10 cm3 d'acétate d'éthyle bouillant. On agite 1 heure à 0¤C et essore. On obtient 0,010 g de maléate attendu. F = 188¤C.

| Analyse : $C_{17}H_{18}N_2O_3$, $C_4H_4O_4$ | | | |
|---|---|---|---|
| Calculé<br>Trouvé | C% 60,86<br>60,8 | H% 5,35<br>5,2 | N% 6,76<br>6,6 |

**Préparation : Chlorure d'acide 2-bromo 4-méthyl butane dioïque.**

A une solution de 13,3 g d'acide 2-bromo 4-méthyl butane dioïque dans 133 cm3 de chloroforme, on ajoute 14,6 cm3 de chlorure de thionyle et agite 1 heure au reflux. On distille le chloroforme et le chlorure de thionyle en excès sous pression réduite et recueille le chlorure d'acide attendu utilisé tel quel.

**Exemple 12 : (14alpha, 16alpha) (±) 14,15-dihydro 11-méthoxy 20,21-dinor 17-oxaéburnaménin-14-ol.**

A une solution de 1,58 g de roduit obtenu au stade D de l'exemple 11 dans 30 cm3 de tétrhydrofuranne, on ajoute en 15 minutes à 0¤/+5¤C 11,2 cm3 d'une solution - 2,3M de diéthyldihydroaluminium de sodium dans le toluène. On agite 1 heure 30 minutes à 0¤/+5¤C puis hydrolyse le milieu par ajout de 20 cm3 de tétrahydrofuranne à 20% d'eau. On agite 2 heures à température ambiante, filtre l'insoluble et amène le filtrat à sec sous pression réduite. On chromatographie le résidu sur silice (éluant : chlorure de méthylène-acétone 1-1) et obtient 0,600 g (isomère B, OH équatorial), F = 202¤C, et 0,440 g (isomère A attendu, OH axial), F = 220¤C.

| Analyse : $C_{17}H_{20}N_2O_3$ | | | |
|---|---|---|---|
| Calculé<br>Trouvé | C% 67,98<br>67,9 | H% 6,71<br>6,9 | N% 9,33<br>9,2 |

**Exemple 13 : Maléate acide de (16alpha) (±) 11-méthoxy 20, 21-dinor 17-oxaéburnaménine.**

**Stade A :** (16alpha) (±) 11-méthoxy 20,21-dinor 17-oxaéburnaménine.

A une solution de 0,58 g de produit B obtenu à l'exemple 12 (OH équatorial) dans 20 cm3 de toluène, on ajoute 30 mg d'acide paratoluènesulfonique et chauffe pendant 1 nuit au reflux. On distille à sec sous pression réduite et chromatographie le résidu sur silice (éluant : acétate d'éthylechlorure de méthylène 8-2), on obtient 0,43 g de produit recherché. F = 104¤C.

Spectre IR ($CHCl_3$)

pas d'OH
-C=C- : 1663 $cm^{-1}$
aromatiques : 1620, 1610, 1568, 1486 $cm^{-1}$
Bandes de Bohlmann.

**Stade B :** Maléate acide de (l6alpha) (+) 11-méthoxy 20,21-dinor 17-oxaéburnaménine.

On dissout 0,38 g de produit obtenu au stade A dans 10 cm3 d'acétate d'éthyle, on ajoute une solution de 0,156 g d'acide maléique dans 5 cm3 d'acétate d'éthyle bouillant. On agite 30 minutes à température ambiante et essore. On obtient 0,53 g de produit brut que l'on recristallise une première fois dans 100 volumes d'acétate d'éthyle puis une seconde fois dans 100 volumes d'acétate, on obtient 0,32 g de produit attendu. F = 185¤C.

| Analyse : $C_{21}H_{22}N_2O_6$ | | | |
|---|---|---|---|
| Calculé<br>Trouvé | C% 63,31<br>63,2 | H% 5,56<br>5,7 | N% 7,03<br>6,9 |

**Exemple 14 : Maléate acide de (16alpha) (±) 10-méthyl 20, 21-dinor 17-oxaéburnaménin-14 (15H)-one.**

**Stade A :** [2-[(5-méthyl 1H-indol-3-yl) éthyl] amino] acétate d'éthyle.

On opère comme au stade A de l'exemple 11 en utilisant 21,5 g de 5-méthyl tryptamine et en opérant 24 heures au reflux au lieu de 24 heures à température ambiante. On obtient 13,2 g de produit recherché.

Spectre IR ($CHCl_3$)

>=0 ester : 1734 $cm^{-1}$
NH indole : 3481 $cm^{-1}$
aromatique : 1626, 1583, 1580 $cm^{-1}$

**Stade B :** 2-[2-[(5-méthyl 1H-indol-3-yl) éthyl] amino] éthanol.

A une solution de 13 g de produit obtenu au stade A dans 260 cm3 de tétrahydrofuranne, on ajoute en 20 minutes à 0¤/+5¤C 300 cm3 de triéthylhydroborure de lithium en solution 1M dans le tétrahydrofuranne. On agite pendant 2 heures à température ambiante puis hydrolyse pendant 1 heure après ajout de 100 cm3 de tétrahydrofuranne à 20% d'eau. On évapore à sec sous pression réduite et reprend le résidu avec 250 cm3 d'acétate d'éthyle et 125 cm3 d'eau. On amène à pH 7 avec de l'acide chlorhydrique N. Après

décantation, on réextrait la phase aqueuse avec deux fois 100 cm3 d'acétate d'éthyle et concentre à sec sous pression réduite. On chromatographie le résidu (10 g) sur silice (éluant : chlorure de méthylèneméthanol-triéthylamine 8-1-1) et obtient 4,4 g de produit attendu.

Spectre RMN 60 MHz en $H_3$ $CDCl_3$ ppm

1,49 :

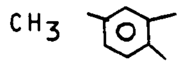

1,60 à 1,90 : N-$CH_2$-$CH_2$-O
2,20 (m) : N-$\underline{CH_2}$-$CH_2$-O
1,79 : -C-$\overline{CH_2}$-$\overline{CH_2}$-N
4,10 à 4,50 : aromatiques
5,09 : proton mobile.

**Stade C** : [4-[2-(5-méthyl 1H-indol-3-yl) éthyl] 3-oxo 2-morpholinyl] acétate de méthyle.

Une solution de chlorure d'acide 2-bromo 4-méthyl butane dioïque (préparée comme indiqué à la préparation 1 à partir de 4,25 g d'acide) dans 20 cm3 de chloroforme est introduite en 10 minutes à température ambiante dans un mélange de 4 g de produit obtenu au stade B, 80 cm3 de chloroforme et 25 g de carbonate de potassium dans 40 cm3 d'eau. On agite 18 heures, décante, réextrait avec du chloroforme et évapore à sec sous pression réduite. On obtient 7 g de résidu que l'on dissout dans 70 cm3 de tétrahydrofuranne, on ajoute 7 g de carbonate de potassium et agite 16 heures au reflux. On filtre l'insoluble et concentre le filtrat à sec sous pression réduite. On chromatographie le résidu (7 g) sur silice (éluant : acétate d'éthyle-chlorure de méthylène 8-2) et obtient 4 g de produit recherché. F = 138 °C.

Spectre IR ($CHCl_3$)

NH indole : 3480 $cm^{-1}$

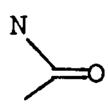

: 1649 $cm^{-1}$

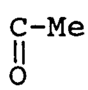

: 1736 $cm^{-1}$
méthyl de l'ester : 1439 $cm^{-1}$

**Stade D** : (16alpha) (±) 10-méthyl 20,21-dinor 17-oxaéburnaménin-14 (15H)-one.

On opère comme au stade D de l'exemple 11 à partir de 2,25 g de produit obtenu au stade C et dans l'oxychlorure de phsophore pur. On obtient 1,53 g de produit attendu. F = 196 °C.

Spectre IR ($CHCl_3$)

NH indole : non détecté
>C = O : 1707 $cm^{-1}$
aromatique : 1656 $cm^{-1}$
Bandes de Bohlmann.

Stade E : Maléate acide de (16alpha) (±) 10-méthyl 20,21-dinor 17-oxaéburnaménin-14 (15H)-one.

On opère comme au stade E de l'exemple 11 à partir de 0,5 g du produit obtenu au stade D. On obtient 0,62 g de produit recherché. F = 200¤C.

| Analyse : $C_{21}H_{22}N_2O_6$ | | | |
|---|---|---|---|
| Calculé<br>Trouvé | C% 63,31<br>63,6 | H% 5,56<br>5,5 | N% 7,03<br>7,0 |

**Exemple 15 : Maléate de (16alpha) (±) 10-méthyl 20,21-dinor 17-oxaéburnaménine.**

**Stade A :** (16alpha) (±) 14,15-dihydro 10-méthyl 20,21-dinor 17-oxaéburnaménin-14-ol.

A une solution de 1,3 g de produit obtenu au stade D de l'exemple 14 dans 26 cm3 de tétrahydrofuranne, on ajoute en 10 minutes à température ambiante 3,9 cm3 d'une solution 2,63M de diéthyldihydroaluminate de sodium dans le toluène. On agite 1 heure à température ambiante puis ajoute 6 cm3 de tétrahydrofuranne à 25% d'eau et agite à nouveau 1 heure. On filtre l'insoluble et évapore le filtrat à sec sous pression réduite. On obtient 1,3 g de mélange de 2 épimères OH axial et équatorial, ce produit brut est utilisé tel quel pour le stade suivant.

**Stade B :** Maléate acide de (16alpha) (±) 10-méthyl 20,21-dinor 17-oxaéburnaménine.

A une solution de 0,39 g de mélange d'isomères (obtenu au stade A) dans 10 cm3 de toluène, on ajoute 20 mg d'acide paratoluènesulfonique et agite 3 heures au reflux. On distille le solvant sous pression réduite et chromatographie le résidu sur silice (éluant : chlorure de méthylène-acétone 1-1) et obtient 0,22 g de produit recherché sous forme de base. F = 130¤C.

Salification :

A une solution de 0,12 g de base dans 5 cm3 d'acétate d'éthyle, on ajoute une solution de 52 mg d'acide maléique dans 2 cm3 d'acétate d'éthyle. On agite 1 heure, essore et obtient 90 mg de produit attendu. F = 166¤C.

| Analyse : $C_{21}H_{22}N_2O_5$ | | | |
|---|---|---|---|
| Calculé :<br>Trouvé : | C% 65,96<br>65,7 | H% 5,80<br>5,8 | N% 7,32<br>7,2 |

**Exemple 16 : Composition pharmaceutique.**

On a préparé des comprimés répondant à la formule suivante :

| | |
|---|---|
| - Produit de l'exemple 1<br>- Excipient pour un comprimé terminé à | 50 mg<br>350 mg. |

(Détail de l'excipient : lactose, talc, amidon, stéarate de magnésium).

**RESULTATS PHARMACOLOGIOUES**

1) Activité analgésique : test de la plaque chaude.

Des souris femelles pesant 22 à 24 g sont placées une par une sur une plaque de cuivre maintenue à 56¤C : la réaction à la douleur se manifeste par le léchage des pattes avant de l'animal ; le temps de cette

réaction est noté et l'on ne retient que les souris réagissant en moins de 8 secondes.

Les animaux sont répartis par groupes homogènes et traités par le produit à étudier administré par voie orale, un groupe ne recevant quele véhicule. Le temps de réaction à la douleur est de nouveau mesuré 30 à 60 minutes après le traitement.

On mesure le pourcentage d'augmentation du temps de réaction en minutes :

| | | |
|---|---|---|
| Témoins | | + 21 |
| Produit de l'exemple 1 | 50 mg/kg<br>100 mg/kg | + 80<br>+ 107 |
| Témoins | | + 24 |
| Produit de l'exemple 9 | 50 mg/kg<br>100 mg/kg | + 17<br>+ 86 |

2) Anoxie asphyxique.

L'épreuve est réalisée chez le rat mâle Sprague Dawley (Charles River) anesthésié à l'éther éthylique, immobilisé (d. tubéécurarine 1 mg/kg I.V.) et ventilés artificielement à l'air. On enregistre l' électrocorticogramme (EcOG)et la pression artérielle. La température rextale et maintenue autour de 36¤C et la capnée entre 35 et 40 torr.

Les produits sont administrés par voie I.V. sous un volume de 1ml/kg 3 minutes avant l'asphyxie obtenue par arrêt de la respiration artificielle. On mesure te temps de latence de disparition de l'EcOG.

A 10 mg/kg, les produits des exemples 1 et 6 ont augmenté respectivement de 23%. et de 34% le temps de latence de disparition de l'EcOG.

**Revendications**

**Revendications pour les Etats contractants suivants : AT, CH, DE, FR, GB, IT, LI, NL**

**1.** Composés de formule (I) :

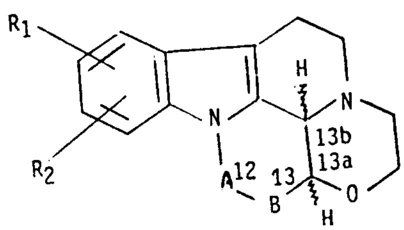

(I)

dans laquelle $R_1$ et $R_2$, identiques ou différents, représentent un atome d'hydrogène, un atome d'halogène, un radical alkyle ou alcoxy renfermant de 1 à 5 atomes de carbone, un radical hydroxy, trifluorométhyle ou nitro et dans laquelle le groupement :

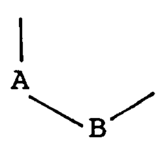

représente soit

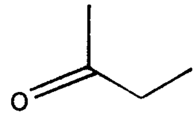

,

soit

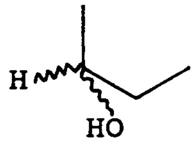,

soit

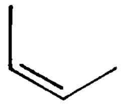,

soit

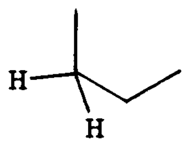,

lesdits produits de formule (I) étant sous toutes les formes isomères possibles racémiques ou optiquement actives, ainsi que les sels d'addition avec les acides minéraux ou organiques desdits produits de formule (I).

**2.** Composés de formule (I) tels que définis à la revendication 1, caractérises en ce que $R_1$ ou $R_2$ représente un atome d'hydrogène, un radical méthyle, éthyle, méthoxy ou éthoxy, un atome de chlore, un radical hydroxy, trifluoro-méthyle ou nitro, sous toutes les formes isomères possibles racémiques ou optiquement actives, ainsi que les sels d'addition avec les acides minéraux ou organiques.

**3.** Composés de formule (I) tels que définis aux revendications 1 et 2, caractérisés en ce que le groupement :

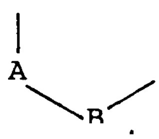.

représente soit

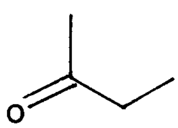,

soit

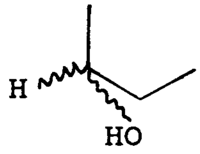,

soit

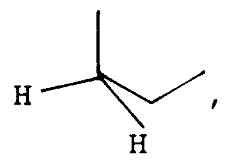

sous toutes les formes isomères possibles racémiques ou optiquement actives, ainsi que les sels d'addition avec les acides minéraux ou organiques.

**4.** L'un quelconque des composés de formule (I) telle que définie à la revendication 1, dont les noms suivent :
- le [12RS (12alpha, 13a alpha, 13b béta)] (±) 2,3,5,6,12,13, 13a,13b-octahydro indolo [3,2,1-de] [1,4] oxazino [2,3,4-ij] [1,5] naphthyridin-12-ol,
- le [12RS (12alpha, 13a béta, 13b alpha)] (±) 2,3,5,6,12,13, 13a,13b-octahydro indolo [3,2,1-de] [1,4] oxazino [2,3,4-ij] [1,5] naphthyridin-12-ol,
- le (13aRS-trans) (±) 2,3,5,6,12,13,13a,13b-octahydro indolo [3,2,1-de] [1,4] oxazino [2,3,4-ij] [1,5] naphthyridine,
- le (13aRS-cis) (±) 2,3,5,6,13a,13b-hexahydro indolo [3,2,1-de] [1,4] oxazino [2,3,4-ij] [1,5] naphthyridin-12 (13H)-one, ainsi que leurs sels d'addition avec les acides.

**5.** Procédé de préparation des composés de formule (I), telle que définie à la revendication 1, caractérisé en ce que l'on fait réagir un composé de formule (II) :

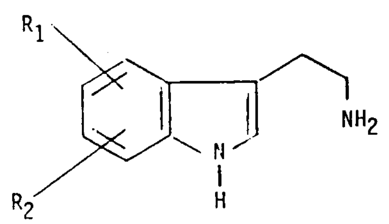

(II)

dans laquelle $R_1$ et $R_2$ ont les significations données à la revendication 1, avec un composé de formule :

$X-CH_2-COOR$

dans laquelle X est un atome d'halogène et R un radical alkyle renfermant 1 ou 2 atomes de carbone, pour obtenir un composé de formule (III) :

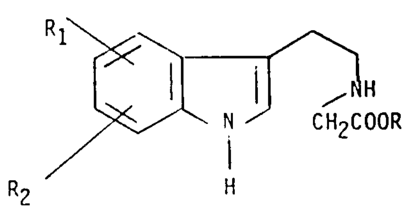

(III)

que l'on réduit pour obtenir un composé de formule (IV) :

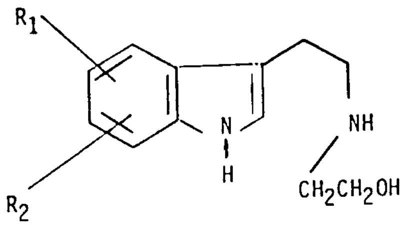

(IV)

que l'on condense avec un chlorure d'acide de formule (VI) :

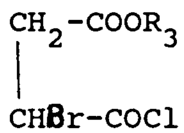

(VI)

dans laquelle $R_3$ est un radical alkyle renfermant 1 ou 2 atomes de carbone, pour obtenir un mélange du produit de formule ($V_A$) :

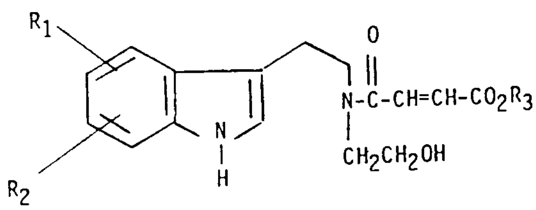

($V_A$)

et du produit de formule (VII) :

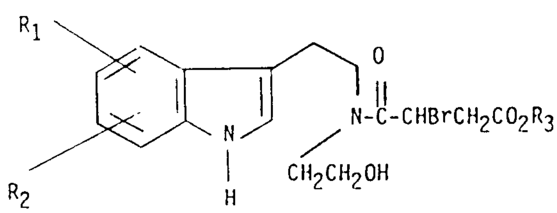

(VII)

produits de formule ($V_A$) et (VII) que l'on cyclise, pour obtenir un produit de formule (VIII) :

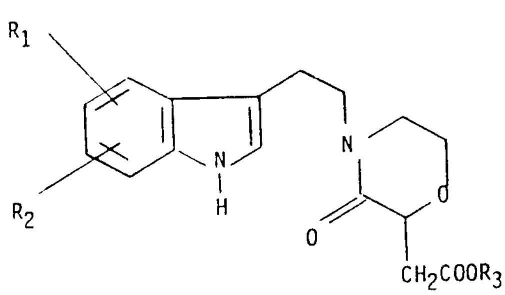

(VIII)

sur lequel l'on fair agir l'oxychlorure de phosphore, pour obtenir le produit de formule ($IX_1$) :

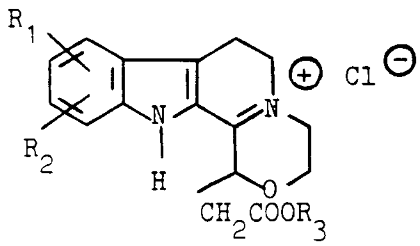

(IX$_1$)

que l'on transforme en perchlorate de formule (IX$_2$) :

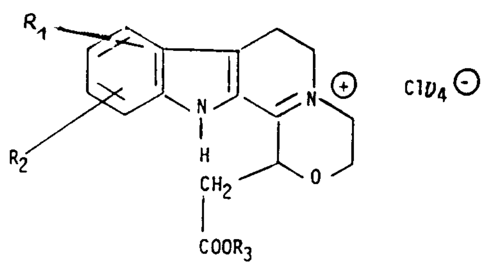

(IX$_2$)

que, soit l'on réduit le composé de formule (IX$_2$) en composé de formule (X) dans laquelles les deux atomes d'hydrogène en positions 13b et 13a sont en position cis :

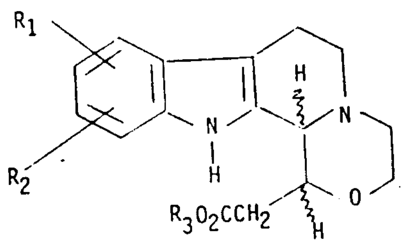

(X)

que l'on cyclise en milieu acide, pour obtenir un composé de formule (IA$_1$) correspondant à un produit de formule (I) dans laquelle :

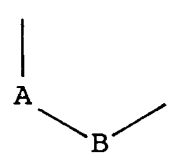

représente

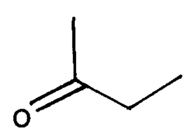

et les deux atomes d'hydrogène en position 13b et 13a sont en position cis soit l'on cyclise en milieu acide le composé de formule (IX$_2$) en composé de formule (XI) :

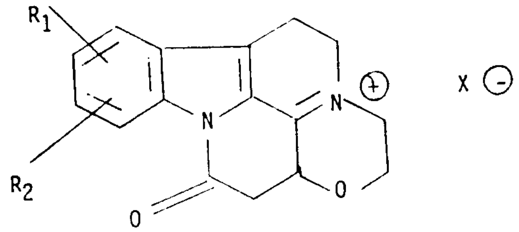

(XI)

$X^-$ étant l'anion provenant de l'acide utilisé et réduit, pour obtenir un composé de formule ($IA_2$) correspondant à un produit de formule (I) dans laquelle

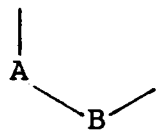

représente

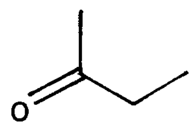

et les deux atomes d'hydrogène en position 13b et 13a sont en positions trans,
lesdits composés de formule ($IA_1$) et ($IA_2$) étant, si désiré, réduits en composés correspondants de formules ($IB_1$) et ($IB_2$), représentant les composés de formule (I) dans laquelle

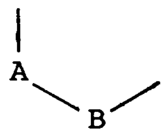

représente

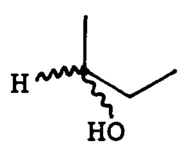

lesdits composés de formules ($IB_1$) et ($IB_2$) étant, si désiré, déshydratés, pour obtenir des composés correspondants de formule ($IC_1$) et ($IC_2$), représentant les composés de formule (I) dans laquelle

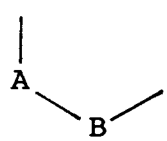

représente

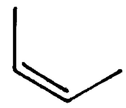

lesdits composés de formules ($IC_1$) et ($IC_2$) étant, si désiré, réduits en composés correspondants de formule ($ID_1$) et ($ID_2$) représentant les composés de formule (I) dans laquelle

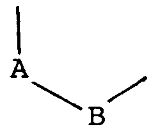

représente

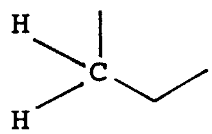

et traite, si désiré, les produits de formule (I) obtenus par un acide minéral ou organique.

**6.** Procédé selon la revendication 5 caractérisé en ce que l'on traite les produits de formule (IV) telle que définie à la revendication 5 avec l'anhydride maléique pour obtenir un produit de formule (V) :

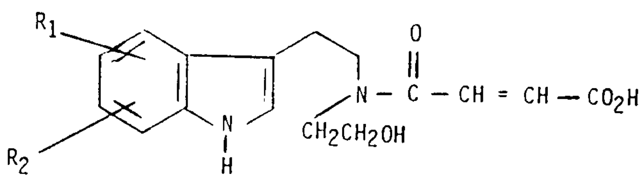

(V)

que l'on estérifie pour obtenir un produit de formule ($V_A$) :

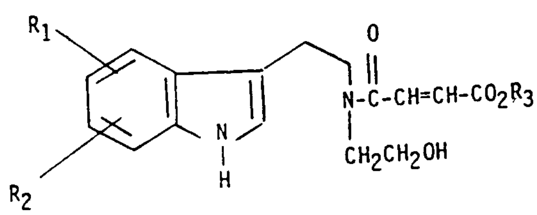

($V_A$)

dans laquelle $R_3$ est un radical alkyle renfermant 1 ou 2 atomes de carbone, que l'on traite ensuite comme indiqué à la revendication 5 pour obtenir les produits de formule (VIII).

**7.** Procédé selon la revendication 5 caractérisé en ce que l'on cyclise les produits de formule ($IX_1$) telle que définie à la revendication 5, en milieu acide ou en milieu basique, pour obtenir un produit de formule (XII) :

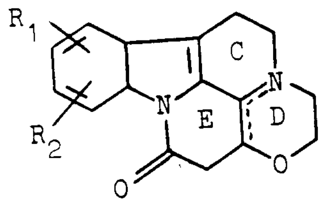

(XII)

dans laquelle le trait pointillé représente une double liaison entre les cycles C et D ou entre les cycles D et E, que l'on réduit, pour obtenir un produit de formule ($IA_2$) telle que définie à la revendication 5, que l'on traite ensuite comme indiqué à la revendication 5 pour obtenir si désiré les produits de formule ($IB_2$).

**8.** A titre de médicaments, les composés tels que définis par la formule (I) de la revendication 1, lesdits produits de formule (I) étant sous toutes les formes isomères possibles racémiques ou optiquement actives, ainsi que les sels d'addition avec les acides minéraux ou organiques pharmaceutiquement acceptables.

**9.** A titre de médicaments, les composés tels que définis par les revendications 2 et 3 pharmaceutiquement acceptables.

**10.** A titre de médicaments, les composés de la revendication 4, pharmaceutiquement acceptables.

**11.** Les compositions pharmaceutiques contenant à titre de principe actif, l'un au moins des médicaments tels que définis à l'une quelconque des revendications 8 à 10.

**12.** Les composés de formules (V), ($V_A$), (VII), (VIII), ($IX_1$), ($IX_2$), (X) et (XII) telles que définies à la revendication 5.

**Revendications pour l'Etat contractant suivant : ES**

**1.** Procédé pour préparer des composés de formule (I) :

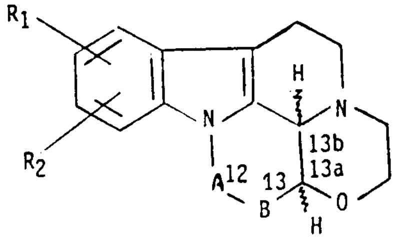

(I)

dans laquelle $R_1$ et $R_2$, identiques ou différents, représentent un atome d'hydrogène, un atome d'halogène, un radical alkyle ou alcoxy renfermant de 1 à 5 atomes de carbone, un radical hydroxy, trifluorométhyle ou nitro et dans laquelle le groupement :

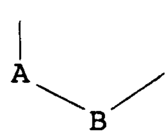

représente soit

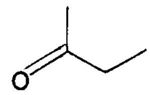

,

soit

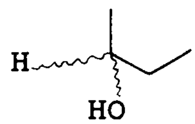

,

soit

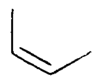

,

soit

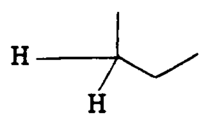

,

lesdits produits de formule (I) étant sous toutes les formes isomères possibles racémiques ou optiquement actives, ainsi que les sels d'addition avec les acides minéraux ou organiques desdits produits de formule (I), caractérisé en ce que l'on fait réagir un composé de formule (II) :

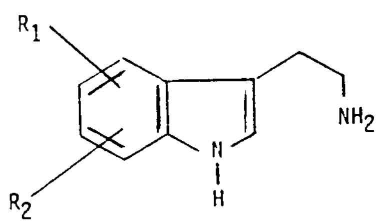

(II)

dans laquelle $R_1$ et $R_2$ ont les significations données à la revendication 1, avec un composé de formule :

X-$CH_2$-COOR

dans laquelle X est un atome d'halogène et R un radical alkyle renfermant 1 ou 2 atomes de carbone, pour obtenir un composé de formule (III) :

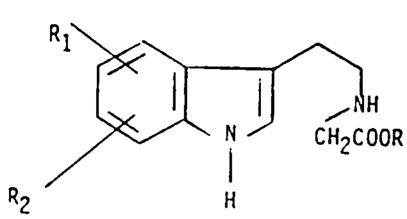

(III)

que l'on réduit pour obtenir un composé de formule (IV) :

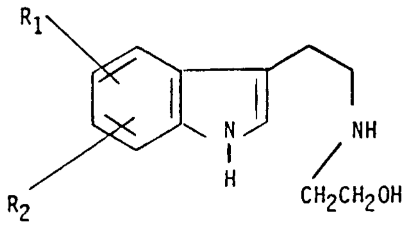

(IV)

que l'on condense avec un chlorure d'acide de formule (VI) :

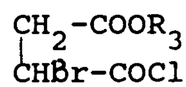

(VI)

dans laquelle $R_3$ est un radical alkyle renfermant 1 ou 2 atomes de carbone, pour obtenir un mélange du produit de formule ($V_A$) :

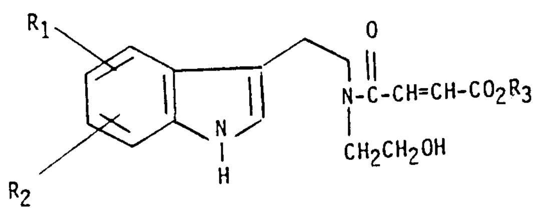

($V_A$)

et du produit de formule (VII) :

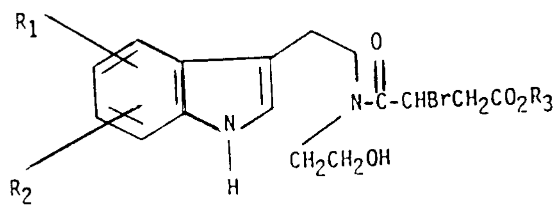

(VII)

produits de formule ($V_A$) et (VII) que l'on cyclise, pour obtenir un produit de formule (VIII) :

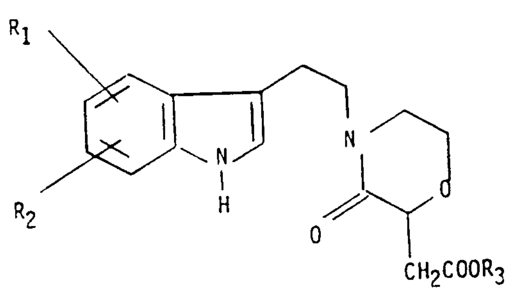

(VIII)

sur lequel l'on fait agir l'oxychlorure de phosphore, pour obtenir le produit de formule ($IX_1$) :

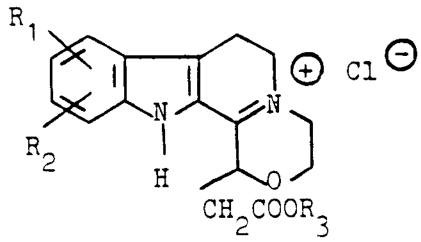

$(IX_1)$

que l'on transforme en perchlorate de formule ($IX_2$) :

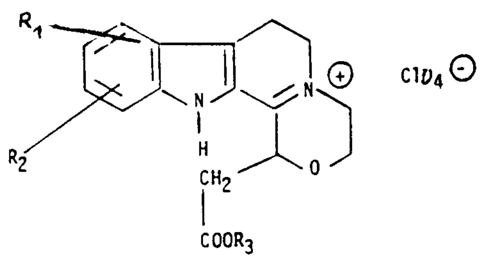

$(IX_2)$

que, <u>soit</u> l'on réduit le composé de formule ($IX_2$) en composé de formule (X) dans laquelles les deux atomes d'hydrogène en positions 13b et 13a sont en position cis :

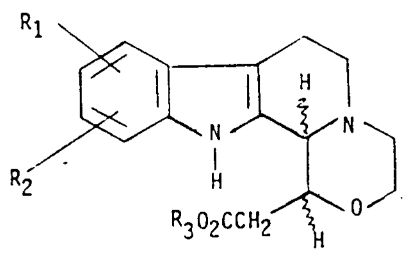

(X)

que l'on cyclise en milieu acide, pour obtenir un composé de formule ($IA_1$) correspondant à un produit de formule (I) dans laquelle :

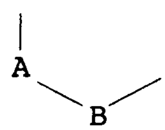

représente

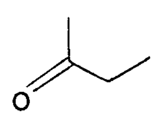

et les deux atomes d'hydrogène en position 13b et 13a sont en position cis <u>soit</u> l'on cyclise en milieu acide le composé de formule ($IX_2$) en composé de formule (XI) :

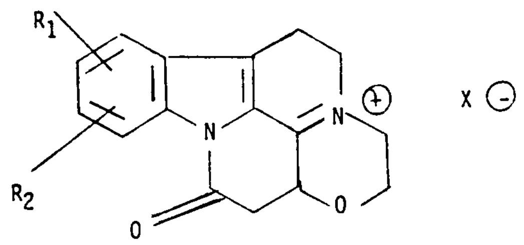

(XI)

$X^{\ominus}$ étant l'anion provenant de l'acide utilisé et réduit, pour obtenir un composé de formule ($IA_2$) correspondant à un produit de formule (I) dans laquelle

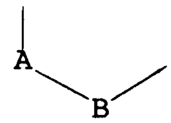

représente

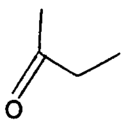

et les deux atomes d'hydrogène en position 13b et 13a sont en positions trans,
lesdits composés de formule ($IA_1$) et ($IA_2$) étant, si désiré, réduits en composés correspondants de formules ($IB_1$) et ($IB_2$), représentant les composés de formule (I) dans laquelle

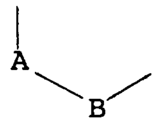

représente

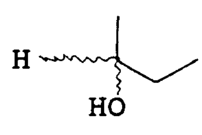

lesdits composés de formules ($IB_1$) et ($IB_2$) étant, si désiré, déshydratés, pour obtenir des composés correspondants de formule ($IC_1$) et ($IC_2$), représentant les composés de formule (I) dans laquelle

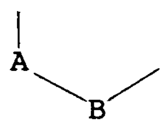

représente

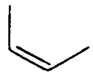

lesdits composés de formules ($IC_1$) et ($IC_2$) étant, si désiré, réduits en composés correspondants de formule ($ID_1$) et ($ID_2$) représentant les composés de formule (I) dans laquelle

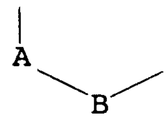

représente

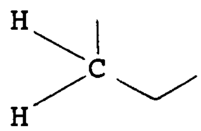

et traite, si désiré, les produits de formule (I) obtenus par un acide minéral ou organique.

**2.** Procédé selon la revendication 1 caractérisé en ce que l'on traite les produits de formule (IV) telle que définie à la revendication 1 avec l'anhydride maléique pour obtenir un produit de formule (V) :

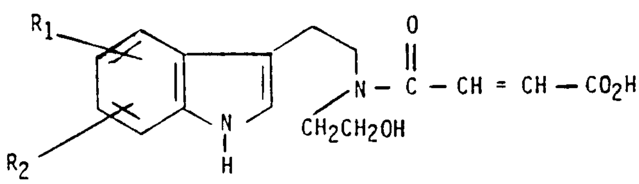

(V)

que l'on estérifie pour obtenir un produit de formule ($V_A$) :

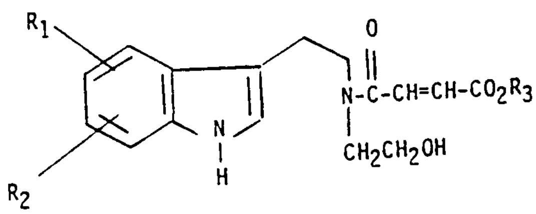

($V_A$)

dans laquelle $R_3$ est un radical alkyle renfermant 1 ou 2 atomes de carbone, que l'on traite ensuite comme indiqué à la revendication 1 pour obtenir les produits de formule (VIII).

**3.** Procédé selon la revendication 1 caractérisé en ce que l'on cyclise les produits de formule ($IX_1$) telle que définie à la revendication 1, en milieu acide ou en milieu basique, pour obtenir un produit de formule (XII) :

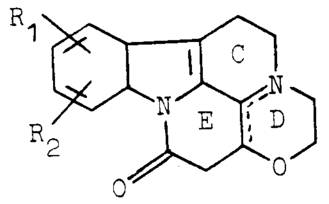

(XII)

dans laquelle le trait pointillé représente une double liaison entre les cycles C et D ou entre les cycles D et E, que l'on réduit, pour obtenir un produit de formule ($IA_2$) telle que définie à la revendication 1, que l'on traite ensuite comme indiqué à la revendication 1 pour obtenir si désiré les produits de formule ($IB_2$).

4. Procédé selon la revendication 1, caractérisé en ce que l'on utilise au départ un composé de formule (II) dans laquelle $R_1$ ou $R_2$ représente un atome d'hydrogène, un radical méthyle, éthyle, méthoxy ou éthoxy, un atome de chlore, un radical hydroxy, trifluoro-méthyle ou nitro.

5. Procédé selon la revendication 1 ou 4, caractérisé en ce que l'on prépare des composés de formule (I) dans laquelle le groupement :

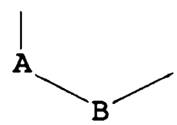

représente soit

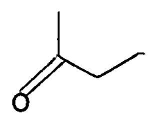

,

soit

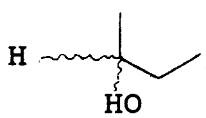

,

soit

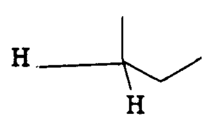

,

sous toutes les formes isomères possibles racémiques ou optiquement actives, ainsi que les sels d'addition avec les acides minéraux ou organiques.

6. Procédé selon la revendication 3 caractérisé en ce que l'on prépare des composés de formule (I) dans laquelle le groupement :

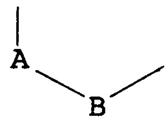

représente soit

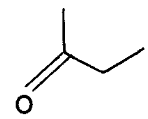 ,

soit

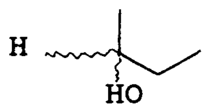 ,

soit

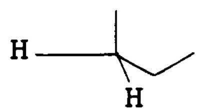 ,

sous toutes les formes isomères possibles racémiques ou optiquement actives, ainsi que les sels d'addition avec les acides minéraux ou organiques.

**7.** Procédé selon la revendication 1 ou 4, caractérisé en ce que l'on prépare l'un quelconque des composés de formule (I) dont les noms suivent :

- le [12RS (12alpha, 13a alpha, 13b béta)] (±) 2,3,5,6,12,13, 13a,13b-octahydro indolo [3,2,1-de] [1,4] oxazino [2,3,4-ij] [1,5] naphthyridin-12-ol,
- le [12RS (12alpha, 13a béta, 13b alpha)] (±) 2,3,5,6,12,13, 13a,13b-octahydro indolo [3,2,1-de] [1,4] oxazino [2,3,4-ij] [1,5] naphthyridin-12-ol,
- le (13aRS-trans) (±) 2,3,5,6,12,13,13a,13b-octahydro indolo [3,2,1-de] [1,4] oxazino [2,3,4-ij] [1,5] naphthyridine,
- le (13aRS-cis) (±) 2,3,5,6,13a,13b-hexahydro indolo [3,2,1-de] [1,4] oxazino [2,3,4-ij] [1,5] naphthyridin-12 (13H)-one, ainsi que leurs sels d'addition avec les acides.

**8.** Procédé selon la revendication 3, caractérisé en ce que l'on prépare l'un quelconque des composés de formule (I) dont les noms suivent :

- le [12RS (12alpha, 13a alpha, 13b béta)] (±) 2,3,5,6,12,13, 13a,13b-octahydro indolo [3,2,1-de] [1,4] oxazino [2,3,4-ij] [1,5] naphthyridin-12-ol,
- le [12RS (12alpha, 13a béta, 13b alpha)] (±) 2,3,5,6,12,13, 13a,13b-octahydro indolo [3,2,1-de] [1,4] oxazino [2,3,4-ij] [1,5] naphthyridin-12-ol,
- le (13aRS-trans) (±) 2,3,5,6,12,13,13a,13b-octahydro indolo [3,2,1-de] [1,4] oxazino [2,3,4-ij] [1,5] naphthyridine,
- le (13aRS-cis) (±) 2,3,5,6,13a,13b-hexahydro indolo [3,2,1-de] [1,4] oxazino [2,3,4-ij] [1,5] naphthyridin-12 (13H)-one, ainsi que leurs sels d'addition avec les acides.

## Claims

**Claims for the following Contracting States : AT, CH, DE, FR, GB, IT, LI, NL**

**1.** The compounds of formula (I):

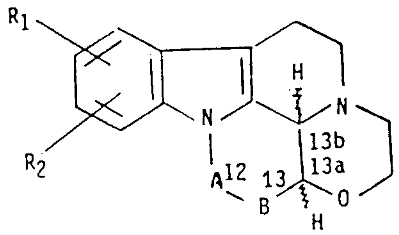

(I)

in which $R_1$ and $R_2$, identical or different, represent a hydrogen atom, a halogen atom, an alkyl or alkoxy radical containing 1 to 5 carbon atoms, a hydroxy, trifluoromethyl or nitro radical and in which the group:

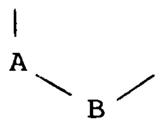

represents either

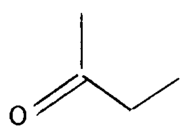

,

or

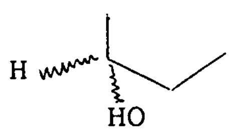

or

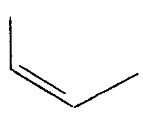

,

or

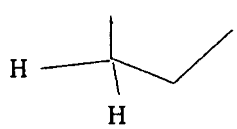

the said products of formula (I) being in all the possible racemic or optically active isomer forms, as well as the addition salts with mineral or organic acids of the said products of formula (I).

2. The compounds of formula (I) as defined in claim 1, characterized in that $R_1$ or $R_2$ represents a hydrogen atom, a methyl, ethyl, methoxy or ethoxy radical, a chlorine atom, a hydroxy, trifluoromethyl or nitro radical, in all the possible racemic or optically active isomer forms, as well as the addition salts with mineral or organic acids.

3. The compounds of formula (I) as defined in claims 1 and 2, characterized in that the group:

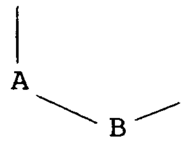

represents either

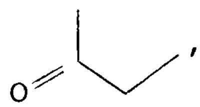

or

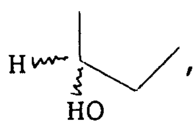

or

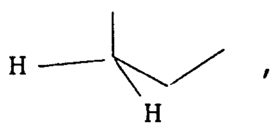

in the all possible racemic or optically active isomer forms, as well as the addition salts with mineral or organic acids.

4. Any one of the compounds of formula (I) as defined in claim 1, whose names follow:
   - [12RS (12alpha, 13a alpha, 13b beta)] (±) 2,3,5,6,12,13, 13a,13b-octahydro indolo [3,2,1-de] [1,4] oxazino [2,3,4-ij] [1,5] naphthyridin-12-ol,
   - [12RS (12alpha, 13a beta, 13b alpha)] (±) 2,3,5,6,12,13, 13a,13b-octahydro indolo [3,2,1-de] [1,4] oxazino [2,3,4-ij] [1,5] naphthyridin-12-ol,
   - (13aRS-trans) (±) 2,3,5,6,12,13,13a,13b-octahydro indolo [3,2,1-de] [1,4] oxazino [2,3,4-ij] [1,5] naphthyridine,
   - (13aRS-cis) (±) 2,3,5,6,13a,13b-hexahydro indolo [3,2,1-de] [1,4] oxazino [2,3,4-ij] [1,5] naphthyridin-12 (13H)-one, as well as their addition salts with acids.

5. Preparation process for the compounds of formula (I), as defined in claim 1, characterized in that a compound of formula (II):

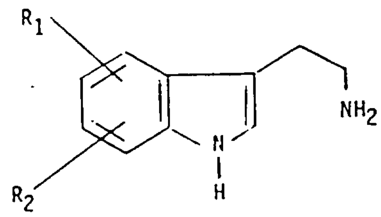

(II)

in which $R_1$ and $R_2$ have the meanings given in claim 1, is reacted with a compound of formula:

$X-CH_2-COOR$

in which X is a halogen atom and R an alkyl radical containing 1 or 2 carbon atoms, in order to obtain a compound of formula (III):

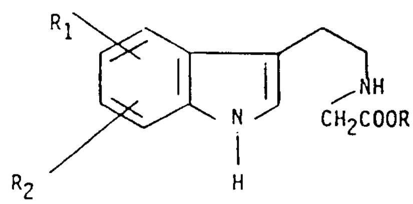

(III)

which is reduced in order to obtain a compound of formula (IV):

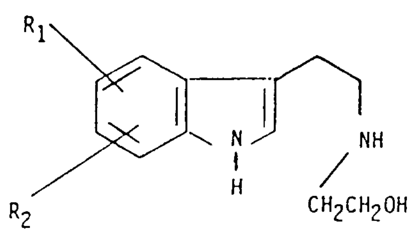

(IV)

which is condensed with an acid chloride of formula (VI):

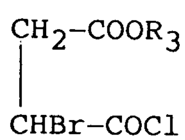

(VI)

in which $R_3$ is an alkyl radical containing 1 or 2 carbon atoms, in order to obtain a mixture of the product of formula ($V_A$):

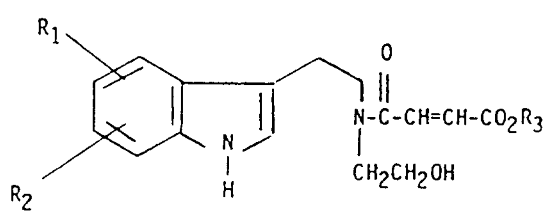

($V_A$)

and the product of formula (VII):

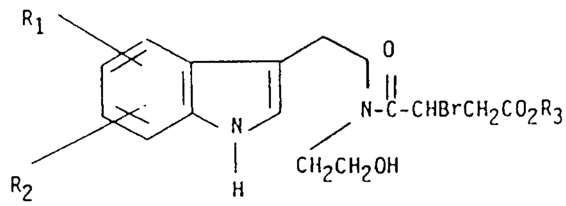

(VII)

which products of formulae ($V_A$) and (VII) are cyclized, in order to obtain a product of formula (VIII):

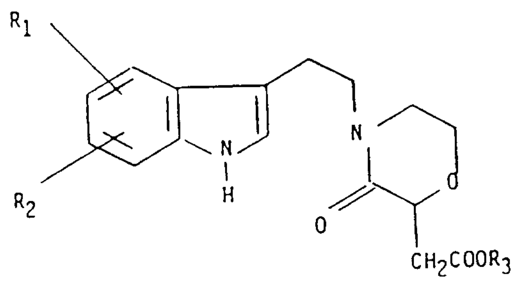

(VIII)

on which phosphorous oxychloride is reacted, in order to obtain the product of formula ($IX_1$):

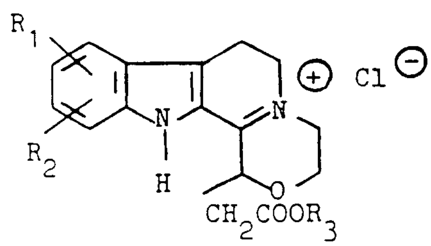

($IX_1$)

which is converted into the perchlorate of formula ($IX_2$):

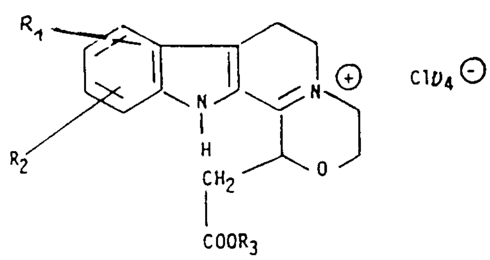

($IX_2$)

either the compound of formula ($IX_2$) is reduced into the compound of formula (X) in which the two hydrogen atoms in positions 13b and 13a are in cis position:

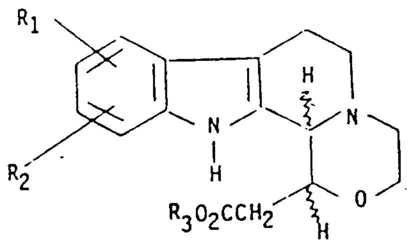

(X)

which is cyclised in an acid medium, in order to obtain a compound of formula ($IA_1$) corresponding to a product of formula (I) in which:

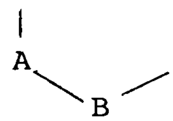

represents

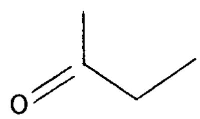

and the two hydrogen atoms in position 13b and 13a are in cis position
or the compound of formula ($IX_2$) is cyclized in an acid medium into the compound of formula (XI):

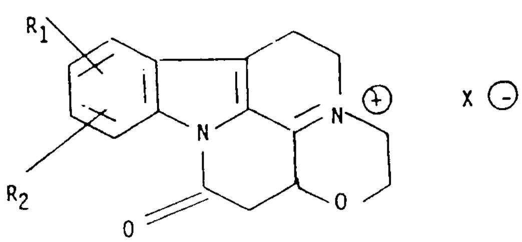

(XI)

$X^-$ being the anion derived from the acid used and reduced, in order to obtain a compound of formula ($IA_2$) corresponding to a product of formula (I) in which

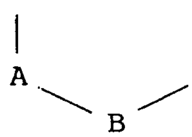

represents

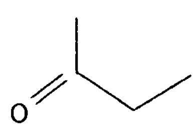

and the two hydrogen atoms in position 13b and 13a are in trans position,

the said compounds of formula ($IA_1$) and ($IA_2$) being, if desired, reduced into the corresponding compounds of formulae ($IB_1$) and ($IB_2$), representing the compounds of formula (I) in which

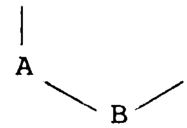

represents

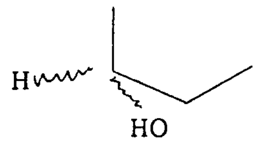

the said compounds of formulae ($IB_1$) and ($IB_2$) being, if desired, dehydrated, in order to obtain the corresponding compounds of formulae ($IC_1$) and ($IC_2$), representing the compounds of formula (I) in which

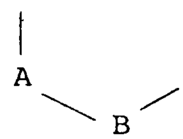

represents

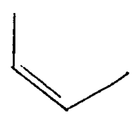

the said compounds of formulae ($IC_1$) and ($IC_2$) being, if desired, reduced into the corresponding compounds of formulae ($ID_1$) and ($ID_2$) representing the compounds of formula (I) in which

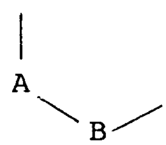

represents

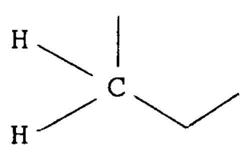

and, if desired, the products of formula (I) obtained are treated with a mineral or organic acid.

**6.** Process according to claim 5 characterized in that the products of formula (IV) as defined in claim 5 are treated with maleic anhydride in order to obtain a product of formula (V):

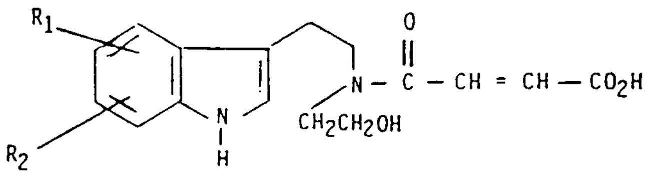

(V)

which is esterified in order to obtain a product of formula ($V_A$):

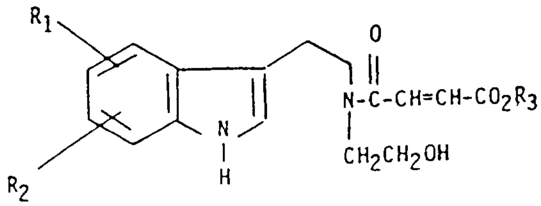

($V_A$)

in which $R_3$ is an alkyl radical containing 1 or 2 carbon atoms, which is then treated as indicated in claim 5 in order to obtain the products of formula (VIII).

**7.** Process according to claim 5 characterized in that the products of formula ($IX_1$) as defined in claim 5 are cyclized in an acid medium or in a basic medium, in order to obtain a product of formula (XII):

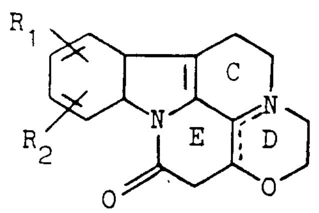

(XII)

in which the dotted line represents a double bond between rings C and D or between rings D and E, which is reduced, in order to obtain a product of formula ($IA_2$) as defined in claim 5, which is then treated as indicated in claim 5 in order to obtain, if desired, the products of formula ($IB_2$).

**8.** As medicaments, the compounds as defined by formula (I) of claim 1, the said products of formula (I) being in all the possible racemic or optically active isomer forms, as well as the addition salts with pharmaceutically acceptable mineral or organic acids.

**9.** As medicaments, the pharmaceutically acceptable compounds as defined by claims 2 and 3.

**10.** As medicaments, the pharmaceutically acceptable compounds of claim 4.

**11.** Pharmaceutical compositions containing as active ingredient, at least one of the medicaments as defined in any one of claims 8 to 10.

**12.** The compounds of formulae (V), ($V_A$), (VII), (VIII), ($IX_1$), ($IX_2$), (X) and (XII) as defined in claim 5.

**Claims for the following Contracting State : ES**

1. Process for the preparation of the compounds of formula (I):

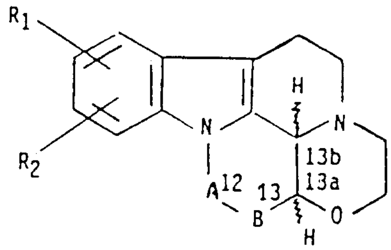

(I)

in which $R_1$ and $R_2$, identical or different, represent a hydrogen atom, a halogen atom, an alkyl or alkoxy radical containing 1 to 5 carbon atoms, a hydroxy, trifluoromethyl or nitro radical and in which the group:

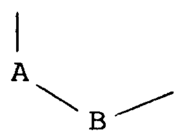

represents either

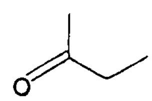

,

or

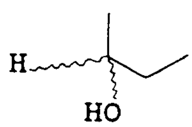

,

or

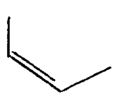

,

or

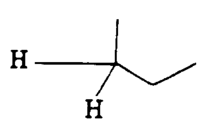

,

the said products of formula (I) being in all the possible racemic or optically active isomer forms, as well as the addition salts with mineral or organic acids of the said products of formula (I), characterized in that a compound of formula (II):

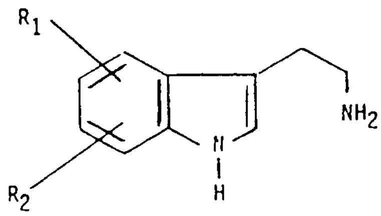

(II)

in which $R_1$ and $R_2$ have the meanings given in claim 1, is reacted with a compound of formula:

$X\text{-}CH_2\text{-}COOR$

in which X is a halogen atom and R an alkyl radical containing 1 or 2 carbon atoms, in order to obtain a compound of formula (III):

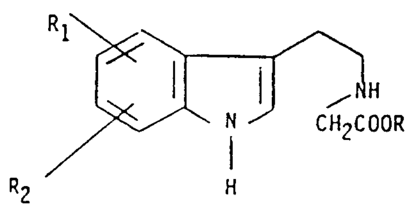

(III)

which is reduced in order to obtain a compound of formula (IV):

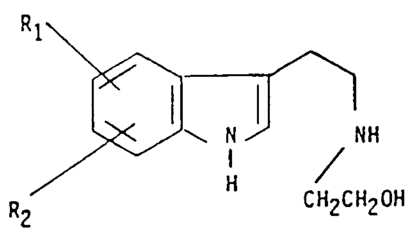

(IV)

which is condensed with an acid chloride of formula (VI):

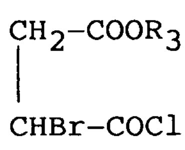

(VI)

in which $R_3$ is an alkyl radical containing 1 or 2 carbon atoms, in order to obtain a mixture of the product of formula ($V_A$):

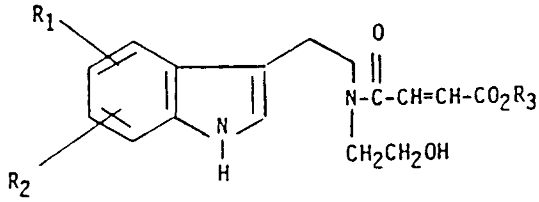

($V_A$)

and the product of formula (VII):

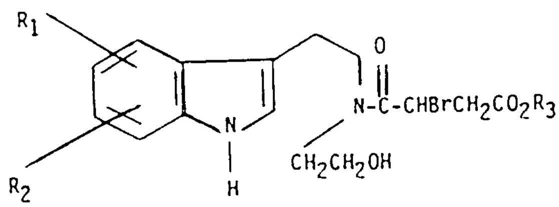

(VII)

which products of formulae ($V_A$) and (VII) are cyclized, in order to obtain a product of formula (VIII):

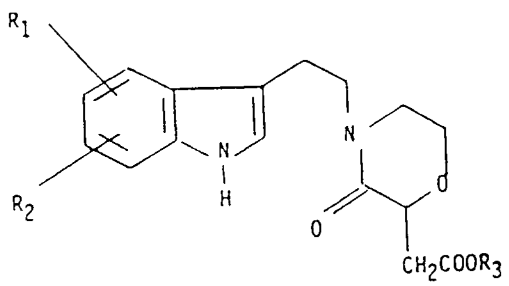

(VIII)

on which phosphorous oxychloride is reacted, in order to obtain the product of formula ($IX_1$):

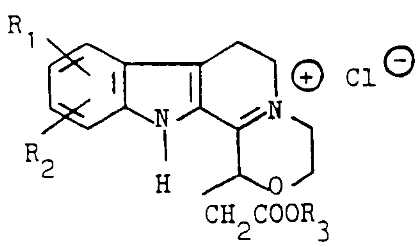

($IX_1$)

which is converted into the perchlorate of formula ($IX_2$):

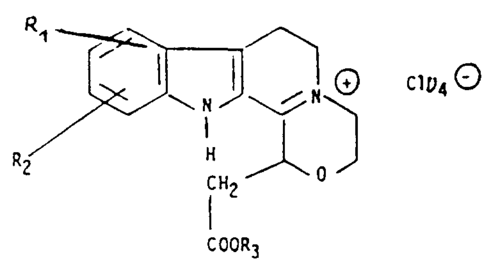

($IX_2$)

either the compound of formula ($IX_2$) is reduced into the compound of formula (X) in which the two hydrogen atoms in positions 13b and 13a are in cis position:

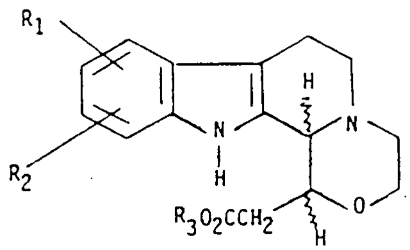

(X)

which is cyclised in an acid medium, in order to obtain a compound of formula ($IA_1$) corresponding to a product of formula (I) in which:

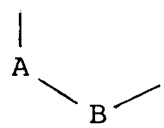

represents

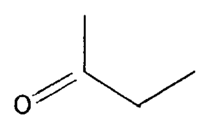

and the two hydrogen atoms in position 13b and 13a are in cis position
or the compound of formula ($IX_2$) is cyclized in an acid medium into compound of formula (XI):

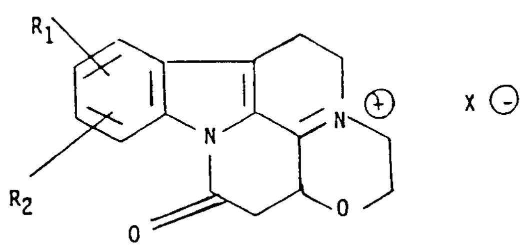

(XI)

$X^-$ being the anion derived from the acid used and reduced, in order to obtain a compound of formula ($IA_2$) corresponding to a product of formula (I) in which

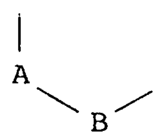

represents

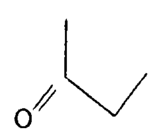

and the two hydrogen atoms in position 13b and 13a are in trans position,
the said compounds of formulae ($IA_1$) and ($IA_2$) being, if desired, reduced into the corresponding compounds of formulae ($IB_1$) and ($IB_2$), representing the compounds of formula (I) in which

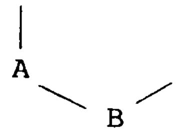

represents

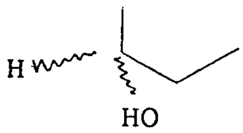

the said compounds of formulae ($IB_1$) and ($IB_2$) being, if desired, dehydrated, in order to obtain the corresponding compounds of formulae ($IC_1$) and ($IC_2$), representing the compounds of formual (I) in which

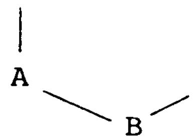

represents

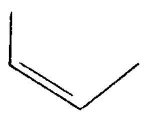

the said compounds of formulae ($IC_1$) and ($IC_2$) being, if desired, reduced into the corresponding compounds of formulae ($ID_1$) and ($ID_2$) representing the compounds of formula (I) in which

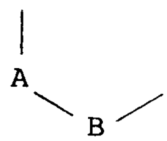

represents

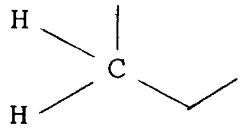

and, if desired, the products of formula (I) obtained are treated with a mineral or organic acid.

**2.** Process according to claim 1 characterized in that the products of formula (IV) as defined in claim 1 are treated with maleic anhydride in order to obtain a product of formula (V):

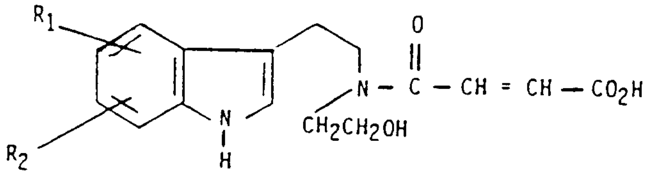

(V)

which is esterified in order to obtain a product of formula ($V_A$):

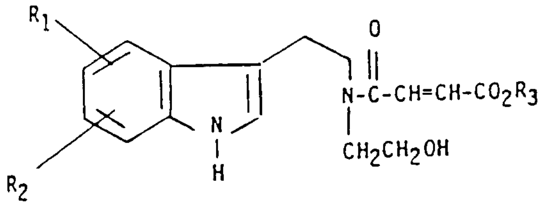

($V_A$)

in which $R_3$ is an alkyl radical containing 1 or 2 carbon atoms, which is then treated as indicated in claim 1 in order to obtain the products of formula (VIII).

**3.** Process according to claim 1 characterized in that the products of formula ($IX_1$) as defined in claim 1 are cyclized in an acid medium or in a basic medium, in order to obtain a product of formula (XII):

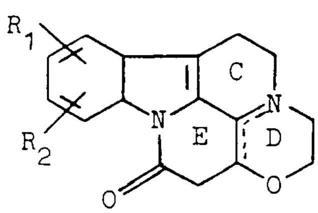

(XII)

in which the dotted line represents a double bond between rings C and D or between rings D and E, which is reduced, in order to obtain a product of formula ($IA_2$) as defined in claim 1, which is then treated as indicated in claim 1 in order to obtain, if desired, the products of formula ($IB_2$).

**4.** Process according to claim 1, characterized in that at the start a compound of formula (II) is used in which $R_1$ or $R_2$ represents a hydrogen atom, a methyl, ethyl, methoxy or ethoxy radical, a chlorine atom, a hydroxy, trifluoromethyl or nitro radical.

**5.** Process according to claim 1 or 4, characterized in that the compounds of formula (I) are prepared in which the group:

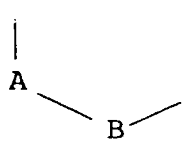

represents either

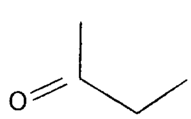 ,

or

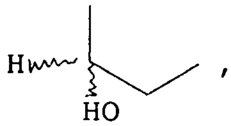

or

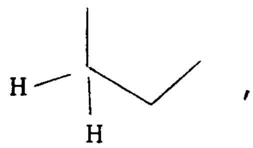

in all the possible racemic or optically active isomer forms, as well as the addition salts with mineral or organic acids.

6. Process according to claim 3 characterized in that the compounds of formula (I) are prepared in which the group:

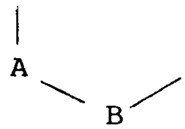

represents either

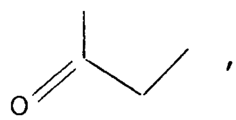

or

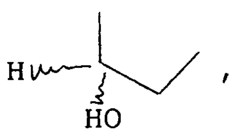

or

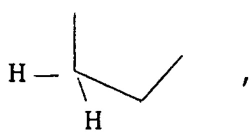

in all the possible racemic or optically active isomer forms, as well as the addition salts with mineral or organic acids.

7. Process according to claim 1 or 4, characterized in that any one of the compounds of formula (I) are prepared the names of which follow:
- [12RS (12alpha, 13a alpha, 13b beta)] (±) 2,3,5,6,12,13, 13a,13b-octahydro indolo [3,2,1-de] [1,4] oxazino [2,3,4-ij] [1,5] naphthyridin-12-ol,

- [12RS (12alpha, 13a beta,13b alpha)] (±) 2,3,5,6,12,13, 13a,13b-octahydro indolo [3,2,1-de] [1,4] oxazino [2,3,4-ij] [1,5] naphthyridin-12-ol,
- (13aRS-trans) (±) 2,3,5,6,12,13,13a,13b-octahydro indolo [3,2,1-de] [1,4] oxazino [2,3,4-ij] [1,5] naphthyridine,
- (13aRS-cis) (±) 2,3,5,6,13a, 13b-hexahydro indolo [3,2,1-de] [1,4] oxazino [2,3,4-ij] [1,5] naphthyridin-12 (13H)-one, as well as their addition salts with acids.

**8.** Process according to claim 3, characterized in that any one of the compounds of formula (I) are prepared, the names of which follow:
- [12RS (12alpha, 13a alpha, 13b beta)] (±) 2,3,5,6,12,13, 13a,13b-octahydro indolo [3,2,1-de] [1,4] oxazino [2,3,4-ij] [1,5] naphthyridin-12-ol,
- [12RS (12alpha, 13a beta, 13b alpha)] (±) 2,3,5,6,12,13, 13a,13b-octahydro indolo [3,2,1-de] [1,4] oxazino [2,3,4-ij] [1,5] naphthyridin-12-ol,
- (13aRS-trans) (±) 2,3,5,6,12,13,13a,13b-octahydro indolo [3,2,1-de] [1,4] oxazino [2,3,4-ij] [1,5] naphthyridine,
- (13aRS-cis) (±) 2,3,5,6,13a,13b-hexahydro indolo [3,2,1-de] [1,4] oxazino [2,3,4-ij] [1,5] naphthyridin-12 (13H)-one, as well as their addition salts with acids.

**Patentansprüche**
**Patentansprüche für folgende Vertragsstaaten : AT, CH, DE, FR, GB, IT, LI, NL**

**1.** Verbindungen der Formel (I)

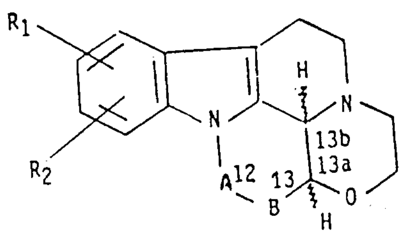

(I)

worin $R_1$ und $R_2$, identisch oder verschieden, ein Wasserstoffatom, ein Halogenatom, einen Alkyl- oder Alkoxyrest mit 1 bis 5 Kohlenstoffatomen, einen Hydroxy-, Trifluormethyl- oder Nitrorest bedeuten, und worin die Gruppe

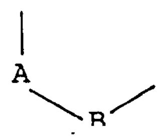

entweder für

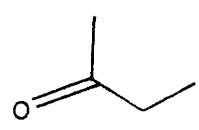

oder

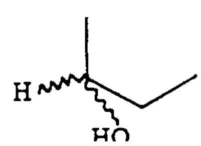

oder

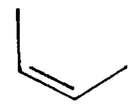

oder

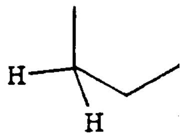

steht, wobei die Produkte der Formel (I) in sämtlichen ihrer möglichen racemischen oder optisch aktiven isomeren Formen vorliegen können, sowie die Additionssalze dieser Produkte der Formel (I) mit Mineral- oder organischen Säuren.

2. Verbindungen der Formel (I), wie in Anspruch 1 definiert, dadurch gekennzeichnet, daß $R_1$ oder $R_2$ ein Wasserstoffatom, einen Methyl-, Ethyl-, Methoxy- oder Ethoxyrest, ein Chloratom, einen Hydroxy-, Trifluormethyl- oder Nitrorest bedeutet, in sämtlichen ihrer möglichen racemischen oder optisch aktiven isomeren Formen, sowie die Additionssalze mit Mineral- oder organischen Säuren.

3. Verbindungen der Formel (I) , wie in den Ansprüchen 1 und 2 definiert, dadurch gekennzeichnet, daß die Gruppe

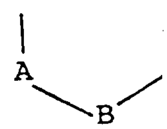

entweder für

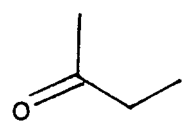

oder

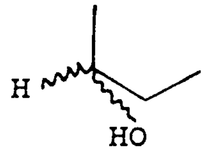

oder

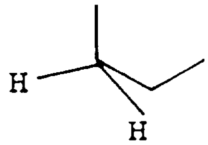

steht, in sämtlichen ihrer möglichen racemischen oder optisch aktiven isomeren Formen, sowie die Additionssalze mit Mineral- oder organischen Säuren.

4. Verbindungen der Formel (I), wie in Anspruch 1 definiert, mit den folgenden Bezeichnungen:
   - [12RS-(12α,13a α,13b β)]-(±)-2,3,5,6,12,13-13a,13b-Octahydroindolo-[3,2,1-de]-[1,4]-oxazino-[2,3,4-ij]-[1,5]-naphthyridin-12-ol,
   - [12RS-(12α,13a β,13b α)]-(±)-2,3,5,6,12,13,13a,13b-Octahydroindolo-[3,2,1-de]-[1,4]-oxazino-[2,3,4-ij]-[1,5]-naphthyridin-12-ol,
   - (13a RS-trans)-(±)-2,3,5,6,12,13,13a,13b-Octahydroindolo-[3,2,1-de]-[1,4]-oxazino-[2,3,4-ij]-[1,5]-naphthyridin,
   - (13a RS-cis)-(±)-2,3,5,6,13a,13b-Hexahydroindolo-[3,2,1-de]-[1,4]-oxazino-[2,3,4-ij]-[1,5]-naphthyridin-12-(13H)-on, sowie deren Additionssalze mit Säuren.

5. Verfahren zur Herstellung der Verbindungen der Formel (I), wie in Anspruch 1 definiert, dadurch gekennzeichnet, daß man eine Verbindung der Formel (II)

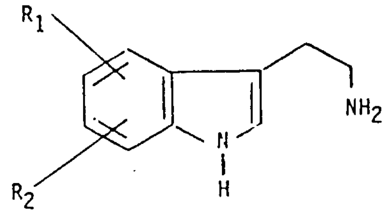

(II)

worin $R_1$ und $R_2$ die in Anspruch 1 angegebene Bedeutung besitzen, mit einer Verbindung der Formel

$X\text{-}CH_2\text{-}COOR$

worin X ein Halogenatom bedeutet, und R für einen Alkylrest mit 1 oder 2 Kohlenstoffatomen steht, umsetzt, um zu einer Verbindung der Formel (III)

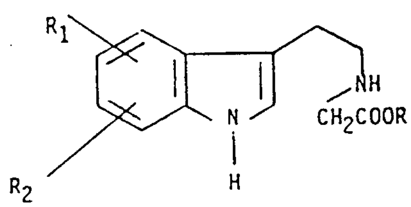

(III)

zu gelangen, die man reduziert, um eine Verbindung der Formel (IV)

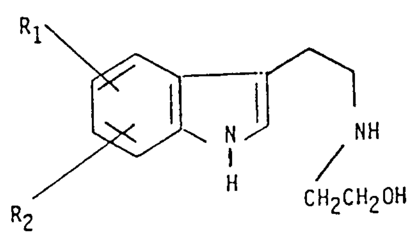

(IV)

zu erhalten, welche man mit einem Säurechlorid der Formel (VI)

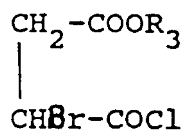

(VI)

worin $R_3$ einen Alkylrest mit 1 oder 2 Kohlenstoffatomen bedeutet, kondensiert, um zu einem Gemisch des Produkts der Formel ($V_A$)

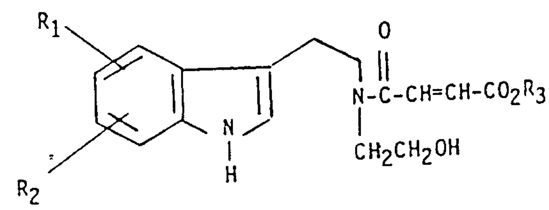

($V_A$)

und des Produkts der Formel (VII)

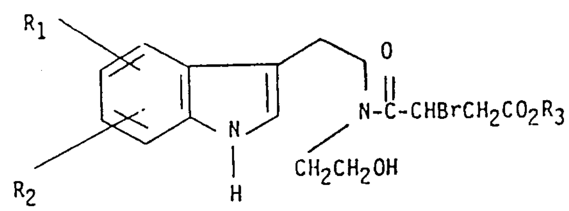

(VII)

zu gelangen, die Produkte der Formel ($V_A$) und (VII) cyclisiert, um ein Produkt der Formel (VIII)

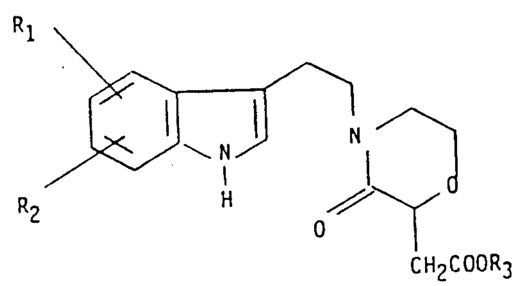

(VIII)

zu erhalten, auf welches man Phosphoroxychlorid reagieren läßt, um zu dem Produkt der Formel ($IX_1$)

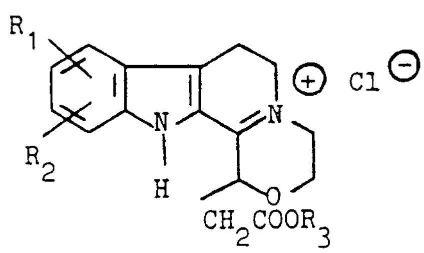

($IX_1$)

zu gelangen, welches man in das Perchlorat der Formel ($IX_2$)

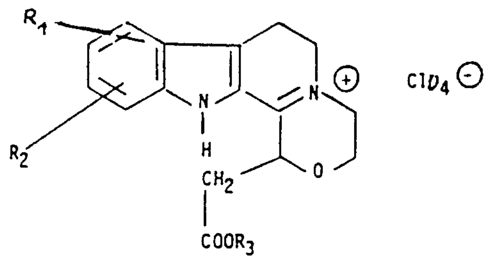

$(IX_2)$

überführt, daß man entweder die Verbindung der Formel ($IX_2$) zu einer Verbindung der Formel (X) reduziert, worin die beiden Wasserstoffatome in den Positionen 13b und 13a in cis-Stellung vorliegen

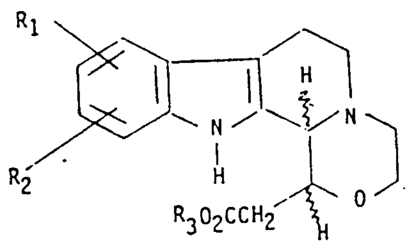

(X)

welche man in saurem Milieu cyclisiert, um zu einer Verbindung der Formel ($IA_1$), entsprechend einem Produkt der Formel (I), worin

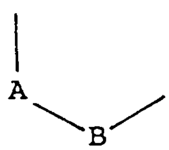

für

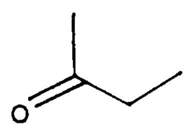

steht, und die beiden Wasserstoffatome in Position 13b und 13a in cis-Stellung vorllegen, zu gelangen, oder die Verbindung der Formel ($IX_2$) in saurem Milieu zu einer Verbindung der Formel (XI)

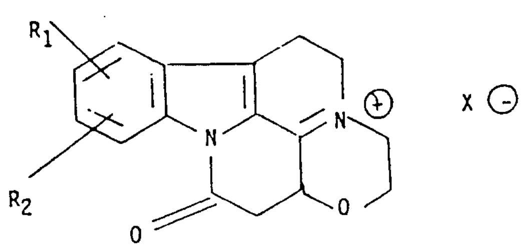

(XI)

worin X- ein Anion ist, das der verwendeten Säure entstammt, cyclisiert und reduziert, um zu einer Verbindung der Formel ($IA_2$),
entsprechend einem Produkt der Formel (I), worin

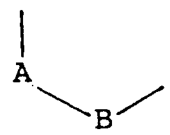

für

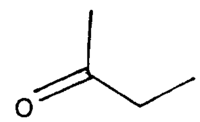

steht, und die beiden Wasserstoffatome in Position 13b und 13a sich in trans-Stellung befinden, zu gelangen,
wobei die Verbindungen der Formel ($IA_1$) und ($IA_2$) gewünschtenfalls zu den entsprechenden Verbindungen der Formeln ($IB_1$) und ($IB_2$) reduziert werden, welche die Verbindungen der Formel (I) , worin

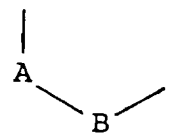

für

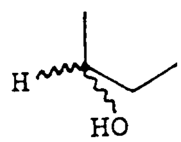

steht, darstellen,
die Verbindungen der Formeln ($IB_1$) und ($IB_2$) gewünschtenfalls dehydratisiert werden, um zu den entsprechenden Verbindungen der Formel ($IC_1$) und ($IC_2$) zu gelangen, die die Verbindungen der Formel (I) darstellen, worin

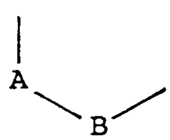

für

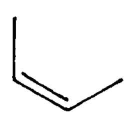

steht,
die Verbindungen der Formeln ($IC_1$) und ($IC_2$) gewünschtenfalls zu entsprechenden Verbindungen der Formel ($ID_1$) und ($ID_2$) reduziert werden, die die Verbindungen der Formel (I) darstellen, worin

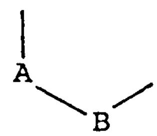

für

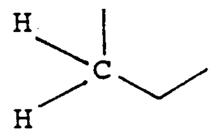

steht, und gewünschtenfalls die erhaltenen Produkte der Formel (I) mit einer Mineral- oder organischen Säure behandelt.

**6.** Verfahren gemäß Anspruch 5, dadurch gekennzeichnet, daß man die Produkte der Formel (IV), wie in Anspruch 5 definiert, mit Maleinsäureanhydrid behandelt, um zu einem Produkt der Formel (V)

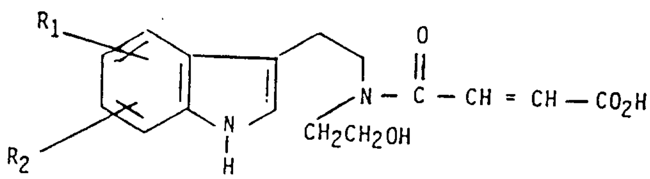

(V)

zu gelangen, welches man verestert, um ein Produkt der Formel ($V_{A}$)

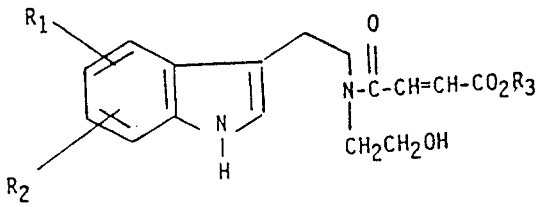

($V_A$)

worin $R_3$ einen Alkylrest mit 1 oder 2 Kohlenstoffatomen bedeutet, zu erhalten, welches man hierauf, wie in Anspruch 5 angegeben, behandelt, um zu den Produkten der Formel (VIII) zu gelangen.

**7.** Verfahren gemäß Anspruch 5, dadurch gekennzeichnet, daß man die Produkte der Formel ($IX_1$), wie in Anspruch 5 definiert, in saurem Milieu oder in basischem Milieu cyclisiert, um ein Produkt der Formel (XII)

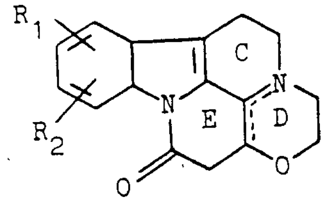

(XII)

worin die gestrichelte Linie eine Doppelbindung zwischen den Ringen C und D oder zwischen den Ringen D und E angibt, zu erhalten, welches man reduziert, um ein Produkt der Formel ($IA_2$), wie in

Anspruch 5 definiert, zu erhalten, welches man hierauf, wie in Anspruch 5 angegeben, behandelt, um gewünschtenfalls die Produkte der Formel ($IB_2$) zu erhalten.

**8.** Als Arzneimittel die Verbindungen der Formel (I) gemäß Anspruch 1, wobei die Produkte der Formel (I) in sämtlichen ihrer möglichen racemischen oder optisch aktiven isomeren Formen vorliegen können, sowie die Additionssalze mit pharmazeutisch verträglichen Mineral- oder organischen Säuren.

**9.** Als Arzneimittel die pharmazeutisch verträglichen Verbindungen, wie in den Ansprüchen 2 und 3 definiert.

**10.** Als Arzneimittel die pharmazeutisch verträglichen Verbindungen gemäß Anspruch 4.

**11.** Pharmazeutische Zusammensetzungen, enthaltend als Wirkstoff zumindest eines der Arzneimittel, wie in einem der Ansprüche 8 bis 10 definiert.

**12.** Die Verbindungen der Formeln (V), ($V_A$), (VII), (VIII), ($IX_1$), ($IX_2$), (X) und (XII), wie in Anspruch 5 definiert.

**Patentansprüche für folgenden Vertragsstaat : ES**

**1.** Verfahren zur Herstellung der Verbindungen der Formel (I)

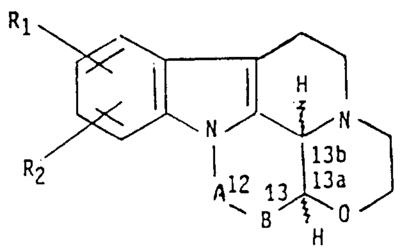

(I)

worin $R_1$ und $R_2$, identisch oder verschieden, ein Wasserstoffatom, ein Halogenatom, einen Alkyl- oder Alkoxyrest mit 1 bis 5 Kohlenstoffatomen, einen Hydroxy-, Trifluormethyl- oder Nitrorest bedeuten, und worin die Gruppe

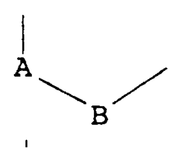

entweder für

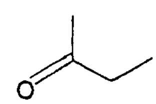

oder

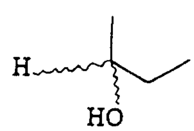

oder

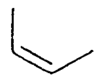

oder

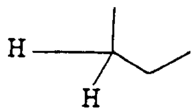

steht, wobei die Produkte der Formel (I) in sämtlichen ihrer möglichen racemischen oder optisch aktiven isomeren Formen vorliegen, sowie der Additionssalze mit Mineral- oder organischen Säuren der Produkte der Formel (I), dadurch gekennzeichnet, daß man eine Verbindung der Formel (II)

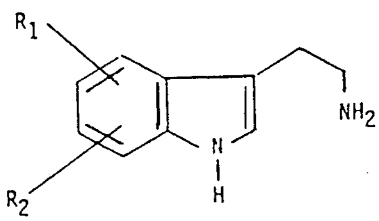

(II)

worin $R_1$ und $R_2$ die in Anspruch 1 angegebene Bedeutung besitzen, mit einer Verbindung der Formel

$X\text{-}CH_2\text{-}COOR$

worin X ein Halogenatom bedeutet, und R für einen Alkylrest mit 1 oder 2 Kohlenstoffatomen steht, umsetzt, um zu einer Verbindung der Formel (III)

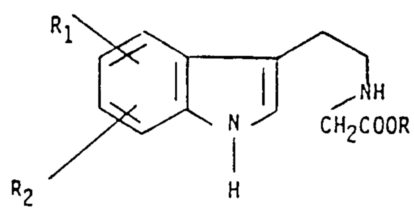

(III)

zu gelangen, die man reduziert, um eine Verbindung der Formel (IV)

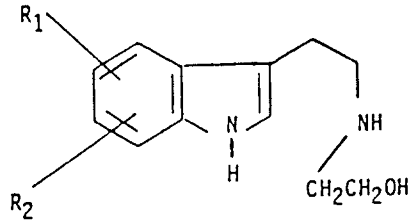

(IV)

zu erhalten, welche man mit einem Säurechlorid der Formel (VI)

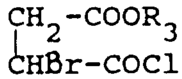

(VI)

worin $R_3$ einen Alkylrest mit 1 oder 2 Kohlenstoffatomen bedeutet, kondensiert, um zu einem Gemisch des Produkts der Formel ($V_A$)

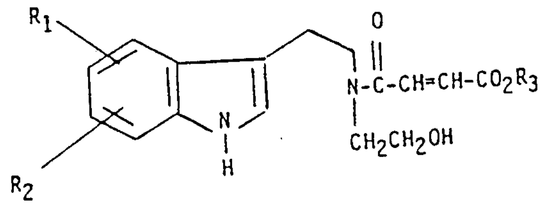

($V_A$)

und des Produkts der Formel (VII)

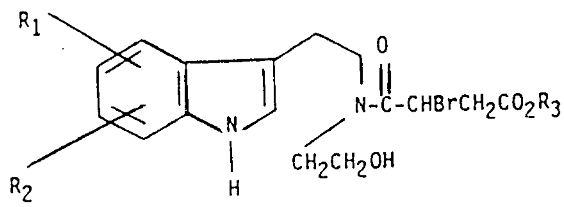

(VII)

zu gelangen, die Produkte der Formel ($V_A$) und (VII) cyclisiert, um ein Produkt der Formel (VIII)

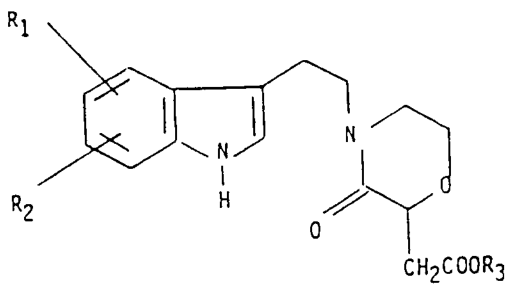

(VIII)

zu erhalten, auf welches man Phosphoroxychlorid reagieren läßt, um zu dem Produkt der Formel ($IX_1$)

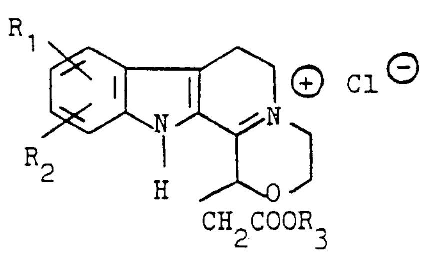

($IX_1$)

zu gelangen, welches man in das Perchlorat der Formel ($IX_2$)

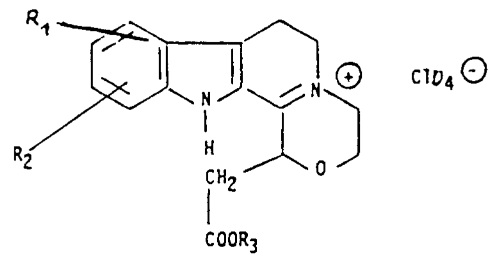

(IX$_2$)

überführt, daß man <u>entweder</u> die Verbindung der Formel (IX$_2$) zu einer Verbindung der Formel (X) reduziert, worin die beiden Wasserstoffatome in den Positionen 13b und 13a in cis-Stellung vorliegen

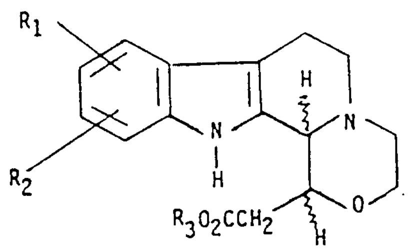

(X)

welche man in saurem Milieu cyclisiert, um zu einer Verbindung der Formel (IA$_1$), entsprechend einem Produkt der Formel (I), worin

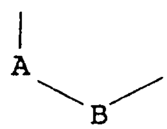

für

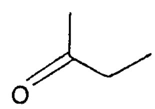

steht und die beiden Wasserstoffatome in Position 13b und 13a in cis-Stellung vorliegen, zu gelangen, <u>oder</u> die Verbindung der Formel (IX$_2$) in saurem Milieu zu einer Verbindung der Formel (XI)

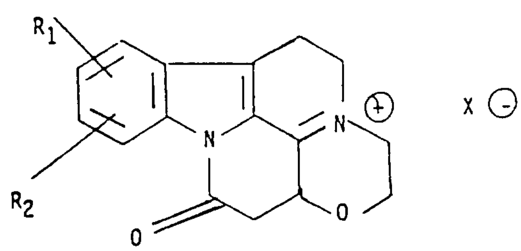

(XI)

worin X- ein Anion ist, das der verwendeten Säure entstammt, cyclisiert und reduziert, um zu einer Verbindung der Formel (IA$_2$),
entsprechend einem Produkt der Formel (I), worin

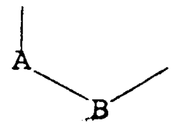

für

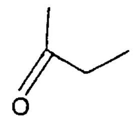

steht, und die beiden Wasserstoffatome in Position 13b und 13a sich in trans-Stellung befinden, zu gelangen,
wobei die Verbindungen der Formel ($IA_1$) und ($IA_2$) gewünschtenfalls zu den entsprechenden Verbindungen der Formeln ($IB_1$) und ($IB_2$) reduziert werden, welche die Verbindungen der Formel (I), worin

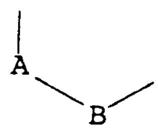

für

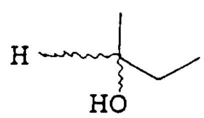

steht, darstellen,
die Verbindungen der Formeln ($IB_1$) und ($IB_2$) gewünschtenfalls dehydratisiert werden, um zu den entsprechenden Verbindungen der Formel ($IC_1$) und ($IC_2$) zu gelangen, die die Verbindungen der Formel (I) darstellen, worin

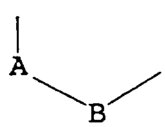

für

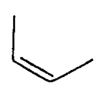

steht,
die Verbindungen der Formeln ($IC_1$) und ($IC_2$) gewünschtenfalls zu entsprechenden Verbindungen der Formel ($ID_1$) und ($ID_2$) reduziert werden, die die Verbindungen der Formel (I) darstellen, worin

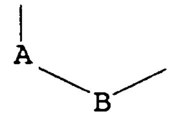

für

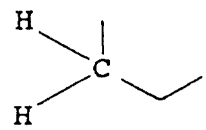

steht, und gewünschtenfalls die erhaltenen Produkte der Formel (I) mit einer Mineral- oder organischen Säure behandelt.

**2.** Verfahren gemäß Anspruch 1, dadurch gekennzeichnet, daß man die Produkte der Formel (IV), wie in Anspruch 1 definiert, mit Maleinsäureanhydrid behandelt, um zu einem Produkt der Formel (V)

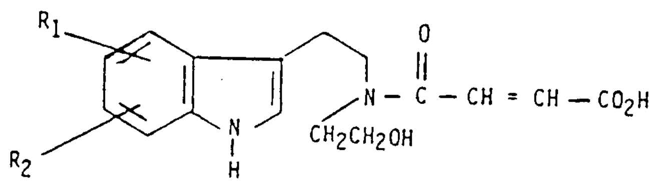

(V)

zu gelangen, welches man verestert, uni ein Produkt der Formel ($V_A$)

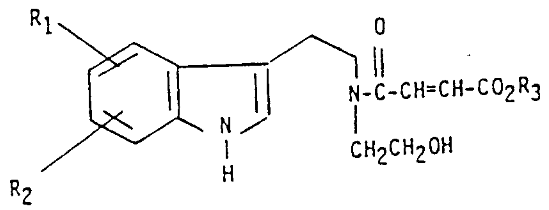

($V_A$)

worin $R_3$ einen Alkylrest mit 1 oder 2 Kohlenstoffatomen bedeutet, zu erhalten, welches man hierauf, wie in Anspruch 1 angegeben, behandelt, um zu den Produkten der Formel (VIII) zu gelangen.

**3.** Verfahren gemäß Anspruch 1, dadurch gekennzeichnet, daß man die Produkte der Formel ($IX_1$), wie in Anspruch 1 definiert, in saurem Milieu oder in basischem Milieu cyclisiert, um ein Produkt der Formel (XII)

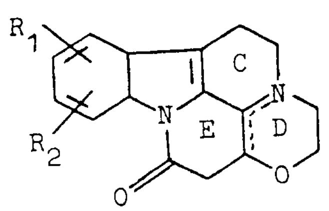

(XII)

worin die gestrichelte Linie eine Doppelbindung zwischen den Ringen C und D oder zwischen den Ringen D und E wiedergibt, zu erhalten, welches man reduziert, um ein Produkt der Formel ($IA_2$), wie in Anspruch 1 definiert, zu erhalten, welches man hierauf, wie in Anspruch 1 angegeben, behandelt, um

gewünschtenfalls die Produkte der Formel ($IB_2$) zu erhalten.

**4.** Verfahren gemäß Anspruch 1, dadurch gekennzeichnet, daß man von einer Verbindung der Formel (II) ausgeht, worin $R_1$ oder $R_2$ ein Wasserstoffatom, einen Methyl-, Ethyl-, Methoxy- oder Ethoxyrest, ein Chloratom, eine Hydroxy-, Trifluormethyl- oder Nitrogruppe bedeutet.

**5.** Verfahren gemäß Anspruch 1 oder 4, dadurch gekennzeichnet, daß man Verbindungen der Formel (I), worin die Gruppe

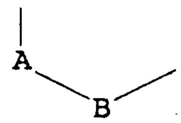

entweder für

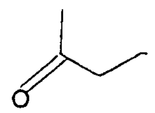

oder

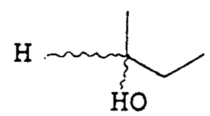

oder

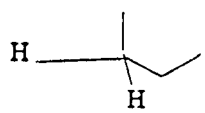

steht, in sämtlichen möglichen racemischen oder optisch aktiven isomeren Formen, sowie die Additionssalze mit Mineral- oder organischen Säuren herstellt.

**6.** Verfahren gemäß Anspruch 3, dadurch gekennzeichnet, daß man die Verbindungen der Formel (I), worin die Gruppe

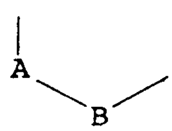

entweder für

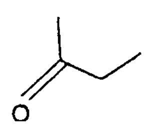

oder oder

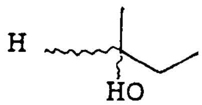

steht, in sämtlichen möglichen racemischen oder optisch aktiven isomeren Formen, sowie die Additionssalze mit Mineral- oder organischen Säuren herstellt.

**7.** Verfahren gemäß Anspruch 1 oder 4, dadurch gekennzeichnet, daß man eine der Verbindungen der Formel (I) mit den folgenden Bezeichnungen:
- [12RS-(12α,13a α,13b β)]-(±)-2,3,5,6,12,13,13a,13b-Octahydroindolo-[3,2,1-de]-[1,4]-oxazino-[2,3,4-ij]-[1,5]-naphthyridin-12-ol,
- [12RS-(12α,13a β,13b α)]-(±)-2,3,5,6,12,13,13a,13b-Octahydroindolo-[3,2,1-de]-[1,4]-oxazino-[2,3,4-ij]-[1,5]-naphthyridin-12-ol,
- (13a RS-trans)-(±)-2,3,5,6,12,13,13a,13b-Octahydroindolo-[3,2,1-de]-[1,4]-oxazino-[2,3,4-ij]-[1,5]-naphthyridin,
- (13a RS-cis)-(±)-2,3,5,6,13a,13b-Hexahydroindolo-[3,2,1-de]-[1,4]-oxazino-[2,3,4-ij]-[1,5]-naphthyridin-12-(13H)-on, sowie deren Additionssalze mit Säuren herstellt.

**8.** Verfahren gemäß Anspruch 3, dadurch gekennzeichnet, daß man eine der Verbindungen der Formel (I) mit den folgenden Bezeichnungen:
- [12RS-(12α,13a α,13b β)]-(±)-2,3,5,6,12,13,13a,13b-Octahydroindolo-[3,2,1-de]-[1,4]-oxazino-[2,3,4-ij]-[1,5]-naphthyridin-12-ol,
- [12RS-(12α,13a β,13b α)]-(±)-2,3,5,6,12,13,13a,13b-Octahydroindolo-[3,2,1-de]-[1,4]-oxazino-[2,3,4-ij]-[1,5]-naphthyridin-12-ol,
- (13a RS-trans)-(±)-2,3,5,6,12,13,13a,13b-Octahydroindolo-[3,2,1-de]-[1,4]-oxazino-[2,3,4-ij]-[1,5]-naphthyridin,
- (13a RS-cis)-(±)-2,3,5,6,13a,13b-Hexahydroindolo-[3,2,1-de]-[1,4]-oxazino-[2,3,4-ij]-[1,5]-naphthyridin-12-(13H)-on, sowie deren Additionssalze mit Säuren herstellt.